# NEW EUROPEAN PATENT SPECIFICATION

(11) **EP 0 974 359 B2**
(45) Date of publication and mention of the opposition decision: **22.04.2009**
(45) Mention of the grant of the patent: 04.12.2002
(21) Application number: 99305664.7
(22) Date of filing: 16.07.1999
(51) Int. Cl.: A61K 38/24, A61K 47/10, A61K 47/18, A61P 15/08

(54) **FSH and FSH variant formulations comprising benzyl alcohol as a preservative**
Benzylalkohol enthaltene FSH und FSH-Varianten Formulierungen
Formulations pharmaceutiques de la FSH et des variantes de FSH comprenant l'alcool benzylique comment agent préservatif.

(30) Priority: 23.07.1998 US 93906 P; 30.07.1998 US 94611 P; 31.07.1998 US 94767 P; 01.09.1998 US 98711 P; 17.09.1998 US 100696 P
(43) Date of publication of application: 26.01.2000
(60) Divisional application: 01130213.0
(73) Proprietor: ARES TRADING S.A., 1170 Aubonne (CH)
(72) Inventor: Hoffmann, James Arthur, Greenwood, Indiana 46143 (US); Lu, Jirong, Indianapolis, Indiana 46326 (US)
(74) Representative: Weiss, Wolfgang

(56) References cited:
- EP-A1- 0 448 146
- EP-A1- 0 853 945
- EP-A2- 0 668 073
- EP-A2- 0 736 303
- EP-A2- 0 891 774
- EP-A2- 0 920 873
- EP-B1- 0 652 766
- WO-A1-93/11788
- WO-A1-97/17087
- GB-A- 839 300
- US-A- 4 659 696
- US-A- 5 162 306
- US-A- 5 661 125
- US-A- 5 733 572
- JÖRGENSEN J.T. ET AL JOURNAL OF PEDIATRIC ENDOCRINOLOGY vol. 7, no. 2, 1994, pages 175 - 180
- MAA Y.-F.; HSU C.C. INTERNATIONAL JOURNAL OF PHARMACEUTICS vol. 140, 1996, pages 155 - 168
- LAM X.M. ET AL PHARMACEUTICAL RESEARCH vol. 14, no. 6, 1997, pages 725 - 729
- PIMPALKHUTE M. ET AL J. RADIOANAL. NUCL. CHEM. LETTERS vol. 103, no. 2, 1986, pages 105 - 116
- GENNARO ET AL REMINGTON'S PHARMACEUTICAL SCIENCES, 18TH EDITION 1990, pages 1549 - 1550
- PAUL HEINZ LIST: 'Arzneiformenlehre', vol. 4TH ED., 1985, WISSENSCHAFTLICHE VERLAGSGESELLSCHAFT MBH, STUTTGART, ISBN 3-8047-0813-7 pages 402 - 407
- WALLHÄUSER K.-H. DEVELOP. BIOL. STANDARD vol. 24, 1974, pages 9 - 28

## Description

This invention relates to new formulations, articles of manufacture and uses of preparations of follicle stimulating hormone (FSH) or follicle stimulating hormone variants (FSH variants) known in the art. The invention also provides advantageous, multi-use and stable solutions and formulations and pharmaceutical products of which have not previously existed for therapeutic use. These formulations and products are particularly useful in therapeutic regimens for increasing serum levels of FSH or FSH variants over a period of treatment. Thus, *inter alia*, the invention fills the need for convenient stable and preserved solutions, formulations and products comprising FSH or FSH variants and using these formulations and products in the treatment of infertility.

FSH is indicated for use in infertility. The patients are administered daily or twice daily intramuscular ("IM") or subcutaneous ("SC") injections with dosage adjusted to response, usually ranging from 75-300 IU/day. The short half-life of FSH makes it necessary that the patients are given once or twice daily injections, extending to several days, depending on their ovarian or testicular response. A more stable formulation of FSH or of a FSH variant would provide improvements for use in therapy.

Although FSH has not been previously administered by approved modes of administration other than by IM or SC, other therapeutic proteins are expected to be administered over an extended number of days. Various delivery methods, including regular SC or IM injections over a period of time, transdermal patches, implants, osmotic pumps, micropumps, cartridges, pulmonary delivery systems, and the like, would be useful, e.g., in facilitating patient compliance, to reducing discomfort, or to facilitating administration. These extended treatment regimens generally require stable solutions or preservatives in the formulation.

Preservatives, in one aspect, prevent or minimize deleterious microbial contamination in the formulation. For conventional, non-protein therapeutics, antimicrobial preservative agents, such as chlorohexidine, phenol, benzyl alcohol, m-cresol, o-cresol, p-cresol, chlorocresol, phenylmercuric nitrate, thimerosal, benzoic acid, alkylparaben (methyl, ethyl, propyl, butyl and the like), benzalkonium chloride, benzethonium chloride, sodium dehydroacetate and thimerosal and various mixtures thereof, are often added to a liquid formulation to ensure sterility during shelf life and/or the multiple use regimen (Akers, MJ, Pharm. Technol. 8, 36-46, 1984; Gennnaro, AR., Remington's Pharmaceutical Sciences, 17th edition., Mack, Easton, PA., 1278-1280, 1985). These preservatives as a class, however, tend to be detrimental to the stability of proteins. For example, a very effective preservative, *m*-cresol, has been reported to generally combine with and denature proteins (Development of Pharmaceutical Parenteral Dosage Forms, Bontempo, ed., Marcel Dekker, Inc., New York, New York, pp. 118-119, 1977). It also presents particular difficulty with the solution stability of hormones, such as human growth hormone (Maa YF and Hsu C, International Journal of Pharmaceutics, 140, pp. 155-168, 1996).

FSH is a member of the heterodimer, glycoprotein hormone family that includes thyroid stimulating hormone (TSH), chorionic gonadotropin (CG), and lutenizing hormone (LH) (Pierce JG and Parsons TF, Annu. Rev. Biochem., 50, 465-495, 1981; Baenziger and Green, Biochem. Biophys. Acta., 947, 287-306, 1988). The members of this family are heterodimers, held together generally by noncovalent interactions between the two different subunits. The human FSH (hFSH) heterodimer consists of (i) a mature 92 amino acid alpha subunit, which also is common to the other human family members (i.e., chorionic gonadotropin ("CG"), leutinizing hormone ("LH") and thyroid stimulating hormone ("TSH")); and (ii) a mature 111 amino acid beta subunit that is unique to FSH (Shome et al., J. Clin. Endocrinol. Metab. 39:187-205 (1974); Shome, et al., J. Prot. Chem, 7:325-339, 1988). The alpha and beta subunits bind non-covalently and, thus, the binding was thought to be more susceptible to protein destabilizing agents.

The native human and other mammalian FSH alpha and beta amino acid sequences and certain variants of these sequences were well known in the art prior to 1982 and cloning and expression of active human and other mammalian FSH in mammalian cells had been accomplished prior to 1985. The common gonadotropin alpha (or FSH alpha) subunit was sequenced from purified protein (Bellisario et al., J. Biol. Chem. 248:6796 (1973); Morgan et al., J. Biol. Chem. 250:5247 (1975)) and later cloned and expressed (Fiddes et al., Nature 281:351 (1979); Nature 286:684 (1981); J. Molec. Appl. Genet. 1:3-18 (1981)). The FSH beta subunit was sequenced from purified protein (Shome et al., J. Clin Endocrinol. Metab. 39:187 (1974); Saxena et al., J. Biol. Chem. 251:993 (1976)); (Sairam et al., Biochem. J. 197:541 (1981); Fujiki et al., Biochem Biophys. Acta 624:428 (1980)). Integrated Genetics reported the recombinant expression of a human CG (Biotechnology Newswatch (p. 3, June 20, 1983); Chemical and Engineering News 61:41 (Nov. 21, 1983); Genetic Technology News 3:9 (Dec. 12, 1983)) and in active form (Biotechnology Newswatch, Jan. 16, 1984)), and they also reported the successful cloning of FSH (Genetic Engineering Newsletter 4:4 (August 10, 1984)) and recombinant FSH produced in mammalian cells in active form (DNA 4:76 (published Jan. 16, 1985)). Amgen also reported the expression of an active bovine LH in CHO cells (Proc. Natl. Acad. Sci. USA 82:7280 (Nov. 1985)).

There is substantial evidence in the literature indicating that heterodimeric protein hormones can dissociate under physiological or acidic conditions (Ryan, R.J., et al., Recent Progr. Hormone Res. 26:105-137; 1970, Strickland, TW and Puett, D, J. Biol. Chem., 257:2954-2960; 1982, Reichert LE and Ramsey RB, J. Biol. Chem., 250:3034-3040; 1975). Intact dimers are essential for biological activity and vital to secretion of FSH (Baenziger JU and Green ED, Biochem. Biophys. Acta, 947:287-306, 1988; Corless, et al., J. Cell Biol., 104:1173-1181, 1987). Attempts to counteract the instability of FSH include those where a single chain molecule is produced, incorporating two subunits into one stable molecule, and those where additional disulfides bonds are created to stabilize the interaction between the two subunits (Sughara T., et al., J. Biol. Chem., 271:10445-10448, 1996; Heikoop J.C., et al., Nature Biotech, 15:658-62, 1997).

Donaldson, U.S. Patent No. 5,162,306, is directed to veterinary compositions comprising FSH and LH. These compositions are shown to be stable in thymol (5-methyl-2(1-methylethyl)phenol). Donaldson reports that thymol is one preservative in the list of preservatives in the U.S.P. XXI that will not damage glycoprotein hormones (U.S. Patent No. 5,162,306) in the disclosed SUPER-OV formulation.

Urinary derived FSH from postmenopausal women (hMG, marketed as Menotropin or Humagon™ by Organon and as urofollitropin or Metrodin™ by Serono) has been used as an injectable for over 30 years for the development of multiple follicles in ovulatory patients participating in Assisted Reproductive Technology (ART) programs and for the induction of ovulation in anovulatory infertile patients. (Fauser BCJM and Van Heusden AM, Endocrine Rev., 18, 71-106, 1997). More recently, CHO cell-derived recombinant human FSH (rhFSH) has become available (Keene J.L., et al., J. Biol. Chem., 264:4769-4752, 1989; Loumaye E., et al., Human Reprod. Update, 1:188-1999, 1995; Olijve W., et al., Mol. Hum. Reprod., 2:361-369, 1996).

Therapeutic FSH (either hMG or rhFSH) is currently supplied in a lyophilized form in ampules of 75 IU/vial and 150 IU/vial with a shelf life of one and a half to two years when stored at 2-25°C. Daily injections with starting doses of 75 IU or 150 IU are recommended for up to ten days to reach steady state concentrations of hFSH that are 1.5-2.5 times higher than that after a single dose administration. This dosing regime yields concentrations necessary for therapeutic efficacy, as FSH acts through a threshold mechanism (Schoemaker J., et al., Ann. NY. Acad. Sci. 687:296-299, 1993). Depending on the patient's response, up to three cycles of treatment with increasing doses of FSH can be used. The patient or the partner is required to reconstitute a new vial of lyophilized material with diluent and administer it immediately after reconstitution (Package insert N1700101A, published in February 1996, for Fertinex™ (urofollitropin for injection, purified) for subcutaneous injection, by Serono Laboratories, Inc., Randolph, MA) on a daily basis. Any unused material is discarded.

Accordingly, there remains a need in the art to increase patient compliance via the development of stable formulations and preserved formulations of FSH or FSH variant proteins, and related articles of manufacture. These stable preparations are especially needed where extended treatments are required or advised, such as fertility treatments with FSH. There is also need to provide an FSH or FSH variant products that can be used and approved for multi-use administration over a period of twenty-four hours or greater. The invention also provides new stable solutions and formulations and preserved solutions and formulations of FSH and FSH variants and the related articles of manufacture that can also be used and approved for use over a period of twenty-four hours or greater.

This invention provides new formulations of FSH or FSH variants, their preparation, and their pharmaceutical or veterinary use in the treatment of fertility disorders and related articles of manufacture.

In one aspect, the invention provides preserved solutions and formulations comprising FSH or a FSH variant and a preservative benzyl alcohol in an aqueous diluent. The solutions and formulations have a pH between 6.8 and 9 and are multi-use formulations in a single solution vial for therapeutic use. Optionally, the preserved solutions and formulations contain a selected buffer and a salt.

This invention also provides an article of manufacture for human pharmaceutical use, comprising packaging material and a single solution vial comprising a solution of FSH or a FSH variant and benzyl alcohol as a preservative, wherein said packaging material comprises a label which indicates that such solution may be held over a period of twenty-four hours or greater for use. The solution has a pH between 6.8 and 9 and is a multi-use formulation for therapeutic use.

These FSH or FSH variant solutions and formulations, articles of manufacture, uses and treatment using a FSH or a FSH variant, with improved or more suitable properties or stability, are useful for infertility treatment in women and/or men. These formulations, articles of manufacture, are additionally suitable for use in injectable and alternative delivery systems, e.g., but not limited to, nasal, pulmonary, transmucosal, transdermal, oral, subcutaneous, intramuscular or parenteral sustained release, liquid formulation. The FSH or a FSH variant solutions and formulations provided may also have increased in vivo potency compared to known commercial products, alone or in combination with at least one additional gonadotropin, by preventing or reducing loss of activity or stability, or by improving any aspect of the effectiveness or desirability of administration, e.g., by at least one of mode, frequency, dosage, comfort, ease of use, biological activity in vitro or in vivo, and the like.

All publications or patents cited herein show the state of the art at the time of the present invention or the filing dates of the related patent applications cited herein.

Follicle stimulating hormone "FSH", whether produced recombinantly or isolated, and follicle stimulating hormone variants "FSH variants" as defined herein are well-known in the art. FSH as used herein refers to the FSH produced as a full length mature protein which includes, but are not limited to human FSH or "hFSH", whether produced recombinantly or isolated from human sources (see Shome B., et al., J. Prot. Chem., 7:325-339, 1988; Saxena B.B. and Rathnam P., J. Biol. Chem., 251:993-1005, 1976; Watkins, et al., DNA, 6:205-212, 1987; Shome B. and Parlow A.F., J. Clin. Endocrinol. Metab., 39(1):203-205, 1974; and Beck, et al., DNA, 4:76, 1985; U.S. 5,405,945, and U.S. 5,639,640). The protein sequence of the human FSH alpha subunit is provided in SEQ ID NO: 5, and the protein sequence of the human FSH beta subunit is given in SEQ ID NO:6. Furthermore, various FSH variants are known or are understood from the art (see Shome, J. Clin. Endocrin. Metab 39:187 (1974); Saxena, J. Biol Chem 251(4):993-1005 (1976); 1978; Sairam et al., Biochem J 197:541 (1981); additionally see Closset Eur. J. Biochem. 86:115-120; Fujiki, J. Biol. Chem. 253:5363-5368 (1978); Sairam, Biochem. J. 197:541-552 (1981). Prior art FSH beta subunits would include the Saxena sequence as well as a genus of sequences implicated in Sairam's discussion of (a) evolutionarily conserved amino acids and (b) well-known and characterized errors in sequencing. Further, those of skill in the art recognize that the substitution of a prior art identified amino acid with (i) a chemically similar amino acid or (ii) an evolutionarily conserved amino acid would have no appreciable affect on the biological activity of an FSH heterodimer comprised of an hFSH beta subunit, thus modified.

In particular, Sairam's commentary on the Saxena hFSH sequence, as well as his discussion of amino acid substitutions identified between functional FSH molecules, defines a genus of FSH beta chain sequences in the prior art. More specifically, the 1981 Sairam publication identifies conserved amino acid sequences referring to publications by Saxena et al., Shome et al., Closset et al., and Fujiki et al. Sairam, Biochem J 197:541, 551 (1981). The prior art (1) evidences a preference for the FSH beta-chain sequence of Saxena over that of Shome; (2) addresses the issue of carboxy-terminal heterogeneity; (3) states that portions of the molecule affected by interspecies differences that are not essential for activity of the hormone and (4) highlights the guidance drawn from homologies between species and between the beta chains of the three, human glycoprotein hormones, FSH, LH and TSH.

C-terminal heterogeneity is reported for all the published sequences except for that of the porcine FSH-s, in which glutamic acid was the only C-terminal residue. For position 27, Saxena assigned one tryptophan residue to this position also found support in the evolutionary conservation demonstrated for a tryptophan at position 24 for FSH-B, among all prior art species. For positions 44 and 46, Saxena shows that, at position 44, the residue should be arginine instead of lysine and, at position 46, lysine instead of arginine. The porcine, equine and ovine sequences also reflected an evolutionary pressure to conserve the arginine at the position 44. The variations at three positions, 21, 22 and 44 involve a structurally conservative or evolutionarily-conserved ("homologous") substitutions, each of which possess bio-activity.

Each of the Sairam, Shome, and Closset references disclose residues isoleucine, serine at positions 21-22, while Saxena discloses leucine, threonine and Fujiki discloses isoleucine, threonine at these positions. Each of these disclosures is not only an evolutionarily conservative substitution, but also a structurally conservative substitution. The variation at position 41 between the aspartic acid disclosed by each of Sairam, Shome, Closset, and Fujiki and the asparagine disclosed by Saxena, Closset and Sairam involves two evolutionarily conserved residues, each of which provide bio-activity. These disclosures of conservative substitutions and evolutionarily conserved substitutions guide the skilled artisan to distinct FSH beta chain variants, within the hFSH-B chain genus.

The FSH variants referred to herein are the carboxy terminal deletions of the beta subunit that are shorter than the full length mature protein of SEQ ID NO:6. Carboxy terminal deletions of the human beta subunit are provided in SEQ IDS NOS: 11, 12, and 13. It is understood that the carboxy terminal variants of the beta chain form dimers with a known alpha subunit to form a FSH variant heterodimer. Additionally, a number of species of FSH are known, including but limited to porcine FSH (SEQ ID NOS: 7 and 8), horse FSH (SEQ ID NOS: 3 and 4), bovine FSH (SEQ ID NOS: 1 and 2), sheep FSH (SEQ ID NOS: 9 and 10), and those cited in Combarnous Y., Endocrine Reviews, 13(4), 670-691, 1992. Therein it is understood that one skilled in the art would be able to make and prepare other carboxy terminal variants from the given species as further provided herein.

FSH heterodimers or FSH variant heterodimers can be produced by any suitable method, such as recombinantly, by isolation or purification from natural sources as may be the case, or by chemical synthesis, or any combination thereof. Non-limiting examples FSH heterodimers and FSH variant heterodimers comprising one alpha subunit and one beta subunit include but are not limited to:
(a) :α-subunit: (SEQ ID NO:1) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
 β -subunit: (SEQ ID NO:2) 
RSCELTNITITVEKEECGFCISINTTWCAGYCYTRDLVYRDPARPNIQKTCTFKEL
VYETVKVPGCAHHADSLYTYPVATECHCSKCDSDSTDCTVRGLGPSYCSFREIKE
(b) :α-subunit: (SEQ ID NO:3) 
FPDGEFTTQDCPECKLRENKYFFKLGVPIYQCKGCCFSRAYPTPARSRKTMLVPKN
ITSESTCCVAKAFIRVTVMGNIKLENHTQCYCSTCYHHKI
 β -subunit: (SEQ ID NO:4) 
NSCELTNITIAVEKEGCGFCITINTTWCAGYCYTRDLVYKDPARPNIQKTCTFKEL
VYETVKVPGCAHHADSLYTYPVATACHCGKCNSDSTDCTVRGLGPSYCSFGDMKE
(c) :α-subunit: (SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -subunit: (SEQ ID NO:6) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMKE
(d) :α-subunit: (SEQ ID NO:7) 
FPDGEFTMQGCPECKLKENKYFSKLGAPIYQCMGCCFSRAYPTPARSKKTMLVPKN
ITSEATCCVAKAFTKATVMGNARVENHTECHCSTCYYHKS
 β -subunit: (SEQ ID NO:8) 
NSCELTNITITVEKEECNFCISINTTWCAGYCYTRDLVYKDPARPNIQKTCTFKEL
VYETVKVPGCAHHADSLYTYPVATECHCGKCDSDSTDCTVRGLGPSYCSFSEMKE
(e) :α-subunit: (SEQ ID NO:9) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
 β -subunit: (SEQ ID NO:10) 
RSCELTNITITVEKEECSFCISINTTWCAGYCYTRDLVYKDPARPNIQKACTFKEL
VYETVKVPGCAHHADSLYTYPVATECHCGKCDRDSTDCTVRGLGPSYCSFSDIRE
(f) :α-subunit: (SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -subunit: (SEQ ID NO:11) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGE
(g) :α-subunit: (SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -subunit: (SEQ ID NO:12) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEM
(h) :α-subunit: (SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -subunit: (SEQ ID NO:13)

NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMK

The use of the term "recombinant" refers to recombinant preparations of FSH or FSH variants through the use of recombinant DNA technology (e.g. Boine et al., Seminars in Reproductive Endocrinology 10, 45-50, 1992, and as generally further provided and exemplified herein). The sequences for genomic and cDNA clones are known for the alpha and beta subunits of several species (Fiddes, J.C., et al., J of Mol. and Applied Genetics, 1:3-18(1981); Esch F.S., et al. DNA 5:363-369(1986); Watkins P.C., et al., DNA 6:205-212(1987); Hirai T., et al., J. Mol. Endrocrinol. 5:147-158(1990); Maurer, R.A., et al., Mol. Endocrinol. 1:717-723(1987); Guzman K., et al., DNA Cell Biol. 10:593-601(1991); Kumar TR, et al., Gene. 1995 Dec 12;166(2):335-6; Kumar TR, et al., Gene. 1995 Dec 12;166(2):333-4. Several of the DNA sequences for alpha and beta subunits are provided as SEQ IDS: 14-20. Moreover, transfection of eucaryotic cells with the DNA sequences encoding a alpha and beta subunit, whether provided on one vector or on two vectors with each subunit having a separate promoter are capable of providing intact dimers, or by other methods understood in the art.

The FSH or a FSH variant used in accordance with the present invention may be produced not only by recombinant means, including from mammalian cell or transgenic preparations, but also may be purified from other biological sources, such as from urinary sources. Acceptable methodologies include those described in Hakola, K. Molecular and Cellular Endocrinology, 127:59-69, 1997; Keene, et al., J. Biol. Chem., 264:4769-4775, 1989; Cerpa-Poljak, et al., Endocrinology, 132:351-356, 1993; Dias, et al., J. Biol. Chem., 269:25289-25294, 1994; Flack, et al., J. Biol. Chem., 269:14015-14020, 1994; and Valove, et al., Endocrinology, 135:2657-2661, 1994, and U.S. Patent 3,119,740.

"Substantially pure," used in reference to a peptide or protein, means separation from other cellular and non-cellular molecules, including other protein molecules. A substantially pure preparation would be about at least 85% pure; preferably about at least 95% pure. A "substantially pure" protein can be prepared by a variety of techniques, well known to the skilled artisan, including, for example, high pressure liquid chromatography (HPLC) and as further understood in the art or demonstrated herein.

The term "administer" or "administering" means to introduce a formulation of the present invention into the body of a patient in need thereof to treat a disease or condition.

The term "patient" means a mammal that is treated for a disease or condition. Patients are of, but not limited to, the following origin, human, ovine, porcine, equine, bovine, rabbit and the like.

The term "alkylparaben" refers to a physiologically tolerated C1-C6 alkyl paraben useful as an antimicrobial agent. Non-limiting examples include at least one methylparaben, ethylparaben, propylparaben, and butylparaben.

The term "aqueous diluent" refers to a liquid solvent that contains water. Aqueous solvent systems may be comprised solely of water, or may be comprised of water plus one or more miscible solvents, and may contain dissolved solutes such as sugars or other excipients. The more commonly-used miscible solvents are the short-chain organic alcohols, such as, methanol, ethanol, propanol, short-chain ketones, such as acetone, and poly Alcohols, such as glycerol.

An "isotonicity agent" is a compound that is physiologically tolerated and imparts a suitable tonicity to a formulation to prevent the net flow of water across cell membranes that are in contact with the formulation. Compounds, such as glycerin, are commonly used for such purposes at known concentrations. Other suitable isotonicity agents include, but are not limited to, amino acids or proteins (e.g., methionine or albumin), salts (e.g., sodium chloride), and sugars (e.g., dextrose, sucrose and lactose).

The term "preservative" refers to a compound or compositions added to a formulation to act as an anti-microbial, anti-fungal, anti-mycoplasmal, anti-viral, anti-prion and/or anti-bacterial agent. A preserved FSH or FSH variant containing formulation of the present invention preferably meets statutory or regulatory guidelines for preservative effectiveness to be a commercially viable multi-use product. The preservative used in the present invention is benzyl alcohol.

The term "phosphate buffer" refers to excipients that contain a phosphate ion. Generally phosphate buffers are prepared from the phosphoric acid, or salt of phosphoric acid, including but not limited to sodium and potassium salts. Several salts of phosphoric acid are known in the art, such as sodium and potassium monobasic, dibasic, and tribasic salts of the acid. Salts of phosphoric acid are also know to occur as hydrates of the occurring salt. Phosphate buffers may cover a wide range of pHs from pH 6.8 to pH 9 and a preferred range to about 8.0. Preferably the formulations of the present invention have pH between about 6.8 and about 7.8, including about pH 7.0, pH 7.2, and 7.4. Preferred ions are the sodium or potassium ions, occurring singularly or together in the solution, as for instance as occurs phosphate buffered saline (PBS). Phosphate saline buffers are well known in the art, such as Dulbecco's Phosphate buffered saline. Salt concentrations in total solution can vary between about 5mM, 9.5mM, 10mM, 50mM, 100mM, 150mM, 200mM, 250mM, and 500mM. Preferably the ion concentration is above 10mM, or above 50mM, or above 100mM.

The term "vial" refers broadly to a reservoir suitable for retaining the FSH and diluent in a contained sterile state. Examples of a vial as used herein include ampules, cartridges, blister packages, or other such reservoir suitable for delivery of the FSH to the patient via pump (including osmotic), catheter, transdermal patch, cartridge, pulmonary, transmucosal, or parenteral delivery. Vials suitable for packaging products for parenteral, pulmonary, transmucosal, or transdermal administration are well-known and recognized in the art.

The term "stability" refers to the physical, chemical, and conformational stability of formulations of FSH of the present invention. Instability of a protein formulation may be caused by chemical degradation or aggregation of the protein molecules to form higher order polymers, by dissociation of the heterodimers into monomers, deglycosylation, modification of glycosylation or any other structural modification that reduces at least one biological activity of an FSH polypeptide included in the present invention.

A "stable" solution or formulation, is one wherein the degree of degradation, modification, aggregation, loss of biological activity and the like, of proteins therein is acceptably controlled, and does not increase unacceptably with time. Stability may be assessed by methods well-known in the art, including measurement of a sample's light scattering, apparent attenuation of light (absorbance, or optical density), size (e.g. by size exclusion chromatography), in vitro or in vivo biological activity and/or properties by differential scanning calorimetry (DSC). Other methods for assessing stability are well known in the art and can also be used according to the present invention.

The term "treating" refers to the administration, follow up, management and/or care of a patient for which FSH administration is desirable for the purpose of follicle or testicular stimulation or any other physiological response regulated by FSH. Treating can thus include, but is not limited to, the administration of FSH for the induction or improvement of sperm or follicular development or for ovulation induction. In addition, treatments for restoring normal spermatogenesis are contemplated in males.

A " salt" is a physiologically-acceptable salt of FSH. Such salts formed between any one or more of the charged groups in the protein and any one or more physiologically-acceptable, non-toxic cations or anions. Organic and inorganic salts include, for example, those prepared from acids such as hydrochloric, sulfuric, sulfonic, tartaric, fumaric, hydrobromic, glycolic, citric, maleic, phosphoric, succinic, acetic, nitric, benzoic, ascorbic, p-toluenesulfonic, benzenesulfonic, naphthalenesulfonic, propionic, carbonic, and the like, or for example, ammonium, sodium, potassium, calcium, or magnesium.

The term "buffer" or "physiologically-acceptable buffer" refers to a compound that is known to be safe for pharmaceutical or veterinary use in formulations and that has the effect of maintaining or controlling the pH of the formulation in the pH range desired for the formulation. Acceptable buffers for controlling pH at a moderately acidic pH to a moderately basic pH include, but are not limited to, such compounds as phosphate, acetate, citrate, arginine, TRIS, and histidine. "TRIS" refers to 2-amino-2-hydroxymethyl-1,3,-propanediol, and to any pharmacologically acceptable salt thereof. Preferable buffers are phosphate buffers with saline or an acceptable salt.

A cDNA or genomic library can be screened using a probe based upon the sequence of a polynucleotide or known nucleic acid to obtain a clone encoding a known FSH sequence. Probes may be used to hybridize with genomic DNA or cDNA sequences to isolate homologous DNA sequences in the same or different organisms. Those of skill in the art will appreciate that various degrees of stringency of hybridization can be employed in the assay; and either the hybridization or the wash medium can be stringent. As the conditions for hybridization become more stringent, there must be a greater degree of complementarity between the probe and the target for duplex formation to occur. The degree of stringency can be controlled by temperature, ionic strength, pH and the presence of a partially denaturing solvent such as formamide. For example, the stringency of hybridization is conveniently varied by changing the polarity of the reactant solution through, for example, manipulation of the concentration of formamide within the range of 0% to 50%. The degree of complementarity (sequence identity) required for detectable binding will vary in accordance with the stringency of the hybridization medium and/or wash medium. The degree of complementarity will optimally be 100%; however, it should be understood that minor sequence variations in the probes and primers may be compensated for by reducing the stringency of the hybridization and/or wash medium.

Methods of amplification of RNA or DNA are well known in the art and can be used according to the present invention without undue experimentation, based on the teaching and guidance presented herein. Methods of selective amplification by PCR allow for the engineering of smaller segments of nucleic acid sequences, such as those that would encode a defined FSH variant beta chain. Such amplification techniques allow adding convenient termination signals, restrictions sites and the like to the amplified sequence.

Known methods of DNA or RNA amplification include, but are not limited to, polymerase chain reaction (PCR) and related amplification processes (see, e.g., U.S. Patent Nos. 4,683,195, 4,683,202, 4,800,159, 4,965,188, to Mullis, et al.; 4,795,699 and 4,921,794 to Tabor, et al; 5,142,033 to Innis; 5,122,464 to Wilson, et al.; 5,091,310 to Innis; 5,066,584 to Gyllensten, et al; 4,889,818 to Gelfand, et al; 4,994,370 to Silver, et al; 4,766,067 to Biswas; 4,656,134 to Ringold) and RNA mediated amplification which uses anti-sense RNA to the target sequence as a template for double-stranded DNA synthesis (U.S. Patent No. 5,130,238 to Malek, et al, with the trade name NASBA), Ausubel, supra; Colligan, supra, Sambrook, supra.

For instance, polymerase chain reaction (PCR) technology can be used to amplify the sequences of polynucleotides and related DNA sequences directly from genomic DNA or cDNA libraries. PCR and other in vitro amplification methods may also be useful, for example, to clone nucleic acid sequences that code for proteins to be expressed as, for example, to obtain any one of the provided FSH or FSH variants, to make nucleic acids to use as probes for detecting the presence of the desired mRNA in samples, for nucleic acid sequencing, or for other purposes. Examples of techniques sufficient to direct persons of skill through in vitro amplification methods are found in Berger, Sambrook, and Ausubel, supra, as well as Mullis, et al., U.S. Patent No. 4,683,202 (1987); and Innis, et al., PCR Protocols A Guide to Methods and Applications, Eds., Academic Press Inc., San Diego, CA (1990). Commercially available kits for genomic PCR amplification are known in the art. See, e.g., Advantage-GC Genomic PCR Kit (Clontech). The T4 gene 32 protein (Boehringer Mannheim) can be used to improve yield of long PCR products.

Nucleic acids required to express any one of the given FSH or FSH variants can also be prepared by direct chemical synthesis by methods such as the phosphotriester method of Narang, et al., Meth. Enzymol. 68:90-99 (1979); the phosphodiester method of Brown, et al., Meth. Enzymol. 68:109-151 (1979); the diethylphosphoramidite method of Beaucage, et al., Tetra. Letts. 22:1859-1862 (1981); the solid phase phosphoramidite triester method described by Beaucage and Caruthers, Tetra. Letts. 22(20):1859-1862 (1981), e.g., using an automated synthesizer, e.g., as described in Needham-VanDevanter, et al., Nucleic Acids Res. 12:6159-6168 (1984); and the solid support method of U.S. Patent No. 4,458,066. Chemical synthesis generally produces a single-stranded oligonucleotide, which may be converted into double-stranded DNA by hybridization with a complementary sequence, or by polymerization with a DNA polymerase using the single strand as a template. One of skill in the art will recognize that while chemical synthesis of DNA is limited to sequences of about 100 bases, longer sequences may be obtained by the ligation of shorter sequences.

As known in the art one can use recombinant expression cassettes to express known encoding nucleic acids for a known FSH or FSH variant. A nucleic acid sequence, for example a cDNA or a genomic sequence encoding a full-length subunit can be used to construct a recombinant expression cassette which can be introduced into a desired host cell. However, it is appreciated in the art that to obtain functional heterodimers one must express both subunits, whether from one plasmid or introduced on separate plasmids. A recombinant expression cassette will typically comprise a polynucleotide operably linked to transcriptional initiation regulatory sequences that will direct the transcription of the polynucleotide in the intended host cell for each subunit. Such methods are well known in the art to express FSH (e.g. CHO cell-derived recombinant human FSH (rhFSH);(Keene J.L., et al., J. Biol. Chem., 264:4769-4752, 1989; Loumaye E., et al., Human Reprod. Update, 1:188-1999, 1995; Olijve W., et al., Mol. Hum. Reprod., 2:361-369, 1996).

Both heterologous and non-heterologous (i.e., endogenous) promoters can be employed to direct expression of the nucleic acids encoding FSH or FSH variant subunits. General production methodologies by recombinant techniques are well known in the art. See, e.g., Sambrook, et al., 1989; Ausubel, et al., 1987-1989.

Encoded polynucleotides for the alpha and beta subunits for FSH or an FSH variant can be joined to a vector containing a selectable marker for propagation in a host. Generally, a plasmid vector (or vectors if alpha and beta subunits are contained on separate expression vectors), is introduced in a precipitate, such as a calcium phosphate precipitate, or in a complex with a charged lipid. If the vector(s) is a virus, it can be packaged in vitro using an appropriate packaging cell line and then transduced into host cells.

The DNA insert for each subunit should be operatively linked to an appropriate promoter, such as the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the mature transcripts expressed by the constructs will preferably include a translation initiating at the beginning and a termination codon (e.g., UAA, UGA or UAG) appropriately positioned at the end of the mRNA to be translated.
Expression vector(s) will preferably include at least one selectable marker. Such markers include, e.g., dihydrofolate reductase or neomycin resistance for eucaryotic cell culture, and tetracycline or ampicillin resistance genes for culturing in E. coli and other bacteria. Representative examples of appropriate hosts include, but are not limited to fungal cells, such as yeast cells; insect cells such as Drosophila S2 and Spodoptera Sf9 cells; animal or mammalian cells such as, but not limited to, CHO, COS, AV-12, HEPG2, NIH3T3 and Bowes melanoma cells; and plant cells, with CHO cells preferred. Appropriate culture mediums and conditions for the above-described host cells are known in the art. Preferred eucaryotic vectors include pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of a vector construct, or vectors, into a host cell can be effected by calcium phosphate transfection, DEAE-dextran mediated transfection, cationic lipid-mediated transfection, electroporation, transduction, infection or other methods. Such methods are described in many standard laboratory manuals, such as Sambrook, supra, Chapters 1-4 and 16-18; Ausubel, supra, Chapters 1, 9, 13, 15, 16.

It is anticipated that FSH or a FSH variant subunits can be expressed in a modified form, such as a fusion protein, and can include not only secretion signals, but also additional heterologous functional regions. For instance, a region of additional amino acids, particularly charged amino acids, can be added to the N-terminus of a polypeptide to improve stability and persistence in the host cell, during purification, or during subsequent handling and storage. Also, peptide moieties can be added to a polypeptide to facilitate purification. Such regions it is anticipated can be removed prior to final preparation of the desired FSH or a FSH variant. Such methods are generally described in many standard laboratory manuals, such as Sambrook, supra, Chapters 17.29-17.42 and 18.1-18.74; Ausubel, supra, Chapters 16, 17 and 18.

Using nucleic acids sequences provided herein or known in the art, one may express the alpha and beta subunits of FSH or a FSH in a recombinantly engineered eucaryotic cell, such as mammalian cells. It is expected that those of skill in the art are knowledgeable in the numerous expression systems available for expression of a nucleic acid encoding a protein that contains two subunits. No attempt to describe in detail the various methods known for the expression of proteins in eucaryotes will be made.

In brief summary, the expression of isolated nucleic acids encoding a known FSH or a FSH variant will typically be achieved by operably linking separately the alpha subunit and the beta subunit DNA or cDNA to a promoter (which is either constitutive or inducible), followed by incorporation into an expression vector(s). Alternative, by inserting the DNA the vector will provide a suitable promoter. The vector(s) can be suitable for replication and integration in eucaryotic cells. Typical expression vectors contain transcription and translation terminators, initiation sequences and promoters useful for regulation of the expression of the DNA encoding a protein of the present invention. To obtain high level expression of a cloned gene, it is desirable to construct expression vectors which contain, at the minimum, a strong promoter to direct transcription, a ribosome binding site for translational initiation, and a transcription/translation terminator. One of skill in the art would recognize that modifications can be made without diminishing its biological activity. Some modifications may be made to facilitate the cloning, expression, or incorporation of the targeting molecules into the genome. Such modifications are well known to those of skill in the art and include, for example, providing conveniently located restriction sites or termination codons or purification sequences.

Alternatively, nucleic acids can be expressed in a host cell by turning on (by manipulation) in a host cell that contains endogenous DNA encoding the desired alpha and beta subunits. Such methods are well known in the art, e.g., as described in US patent Nos. 5,580,734, 5,641,670, 5,733,746, and 5,733,761.

A variety of eucaryotic expression systems such as mammalian cells, are known to those of skill in the art. As explained briefly below, a nucleic acid encoding for the alpha and beta subunit of a known FSH or a FSH variant can be expressed in these eucaryotic systems.

Synthesis of heterologous proteins in yeast is well known. F. Sherman, et al., Methods in Yeast Genetics, Cold Spring Harbor Laboratory (1982) is a well-recognized work describing the various methods available to produce the protein in yeast. Two widely utilized yeast for production of eucaryotic proteins are Saccharomyces cerevisiae and Pichia pastoris. Vectors, strains, and protocols for expression in Saccharomyces and Pichia are known in the art and available from commercial suppliers (e.g., Invitrogen). Suitable vectors usually have expression control sequences, such as promoters, including 3-phosphoglycerate kinase or alcohol oxidase, and an origin of replication, termination sequences and the like as desired.

The sequences encoding the alpha and beta subunits of FSH or a FSH variant can also be ligated to various expression vectors for use in transfecting cell cultures of, for instance, mammalian, insect, or plant origin. Illustrative of cell cultures useful for the production of the peptides are mammalian cells. Mammalian cell systems often will be in the form of monolayers of cells although mammalian cell suspensions may also be used. A number of suitable host cell lines capable of expressing intact proteins have been developed in the art, and include the HEK293, BHK21, and CHO cell lines, with CHO cell lines preferred, such as CHO K1 from Lonza. Expression vectors for these cells can include expression control sequences, such as an origin of replication, a promoter (e.g., preferably the CMV promoter, a HSV tk promoter, EF1 alpha promoter, late or early SV40 promoter, or pgk (phosphoglycerate kinase) promoter), an enhancer (Queen, et al., Immunol. Rev. 89:49 (1986)), and processing information sites, such as ribosome binding sites, RNA splice sites, polyadenylation sites (e.g., an SV40 large T Ag poly A addition site), and transcriptional terminator sequences. Other animal cells useful for production of proteins of the present invention are available, for instance, from the American Type Culture Collection Catalogue of Cell Lines and Hybridomas (7th edition, 1992). Preferred host cells include CHO cells, such as CHO-K1 and preferred vectors include GS vectors, each available, e.g., from Lonza Biologics PLC (Slough, Berkshire, England, UK).

Appropriate vectors for expressing the alpha and beta subunit of FSH or a FSH variant in insect cells are usually derived from the SF9 baculovirus. Suitable insect cell lines include mosquito larvae, silkworm, armyworm, moth and Drosophila cell lines such as a Schneider cell line (See Schneider, J. Embryol. Exp. Morphol. 27:353-365 (1987).

As with yeast, when higher animal or plant host cells are employed, polyadenlyation or transcription terminator sequences are typically incorporated into the vector. An example of a terminator sequence is the polyadenlyation sequence from the bovine growth hormone gene. Sequences for accurate splicing of the transcript may also be included. An example of a splicing sequence is the VP1 intron from SV40 (Sprague, et al., J. Virol. 45:773-781 (1983)). Additionally, gene sequences to control replication in the host cell may be incorporated into the vector such as those found in bovine papilloma virus type-vectors. M. Saveria-Campo, Bovine Papilloma Virus DNA, a Eucaryotic Cloning Vector in DNA Cloning Vol. II, a Practical Approach, D. M. Glover, Ed., IRL Press, Arlington, VA, pp. 213-238 (1985).

FSH or a FSH variant, once expressed, can be isolated from the cells by applying standard protein isolation techniques to the lysates. The monitoring of the purification process can be accomplished by using Western blot techniques or radioimmunoassay of other standard immunoassay techniques.

FSH or a FSH variant, containing an alpha and beta subunit, can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography, size exclusion chromatography, and lectin chromatography. Preferably, high performance liquid chromatography ("HPLC"), cation exchange chromatography, affinity chromatography, size exclusion chromatography, or combinations thereof, are employed for purification. FSH and FSH variants having an alpha and beta subunit include naturally purified products, products of chemical synthetic procedures, and include products produced by recombinant techniques from a eucaryotic host, including, for example, yeast, higher plant, insect and mammalian cells. Depending upon the host employed in a recombinant production procedure, the polypeptides of the present invention can be glycosylated or can be non-glycosylated. Preferred FSH or a FSH variant molecules are glycosylated as would occur in eucaryotic hosts. In addition, polypeptides of the invention can also include an initial modified methionine residue, in some cases as a result of host-mediated processes. Such methods are described in many standard laboratory manuals, such as Sambrook, supra, Chapters 17.37-17.42; Ausubel, supra, Chapters 10, 12, 13, 16, 18 and 20.

FSH or a FSH variant known in the art, include but are not limited to those protein sequences listed in the sequence identification portion of the specification of which are further identified below:

| | | |
|---|---|---|
| SEQ ID NO: 1; | bovine alpha subunit | 96 amino acids |
| SEQ ID NO: 2; | bovine beta subunit | 111 amino acids |
| SEQ ID NO: 3; | equine alpha subunit | 96 amino acids |
| SEQ ID NO: 4; | equine beta subunit | 111 amino acids |
| SEQ ID NO: 5; | human alpha subunit | 92 amino acids |
| SEQ ID NO: 6; | human beta subunit | 111 amino acids |
| SEQ ID NO: 7; | porcine alpha subunit | 96 amino acids |
| SEQ ID NO: 8; | porcine beta subunit | 111 amino acids |
| SEQ ID NO: 9; | ovine alpha subunit | 96 amino acids |
| SEQ ID NO: 10; | ovine beta subunit | 111 amino acids- |
| SEQ ID NO: 11; | human beta variant | 108 amino acids |
| SEQ ID NO: 12; | human beta variant | 109 amino acids |
| SEQ ID NO: 13; | human beta variant | 110 amino acids |

FSH or a FSH variant nucleotide sequence, include but are not limited to those nucleotide sequences which encode an alpha or a beta subunit listed in the sequence identification portion of the specification of which are further identified below:

| | | |
|---|---|---|
| SEQ ID NO:14; | human alpha cDNA | 276 nucleotides |
| | (codes 92 amino acids) | |
| SEQ ID NO:15; | h. beta variant cDNA | 324 nucleotides |
| | (codes 108 amino acids) | |
| SEQ ID NO:16; | h. beta variant cDNA | 327 nucleotides |
| | (codes 109 amino acids) | |
| SEQ ID NO:17; | h. beta variant cDNA | 330 nucleotides |
| | (codes 110 amino acids) | |
| SEQ ID NO:18; | h. beta cDNA | 333 nucleotides |
| | (codes 111 amino acids) | |
| SEQ ID NO:19; | human alpha cDNA | 276 nucleotides |
| | (codes 92 amino acids) | |
| SEQ ID NO:20; | h. beta variant cDNA | 324 nucleotides |
| | (codes 108 amino acids) | |

The DNA of SEQ ID NO:19 and 20 is designed and constructed from ligated oligonucleotides. The differences between SEQ ID NO:19 and SEQ ID NO:14 are one that do not change the encoded amino acid sequence of the alpha subunit protein. Likewise, the differences between SEQ ID NO:20 and SEQ ID NO:15 are ones that do not change the encoded amino acid sequence of the beta variant subunit protein.

The amino acids that make up the proteins and polypeptides of the present invention are often abbreviated. The amino acid designations can be indicated by designating the amino acid by its single letter code, its three letter code, name, or three nucleotide codon(s) as is well understood in the art (see Alberts, B., et al., Molecular Biology of The Cell, Third Ed., Garland Publishing, Inc.,New York, 1994):

| SINGLE LETTER CODE | THREE LETTER CODE | NAME | THREE NUCLEOTIDE CODON(S) |
|---|---|---|---|
| A | Ala | Alanine | GCA, GCC, GCG, |
| | | | GCU |
| C | Cys | Cysteine | UGC, UGU |
| D | Asp | Aspartic acid | GAC, GAU |
| E | Glu | Glutamic acid | GAA, GAG |
| F | Phe | Phenylanine | UUC, UUU |
| G | Gly | Glycine | GGA, GGC, GGG, |
| | | | GGU |
| H | His | Histidine | CAC, CAU |
| I | Ile | Isoleucine | AUA, AUC, AUU |
| K | Lys | Lysine | AAA, AAG |
| L | Leu | Leucine | UUA, UUG, CUA, |
| | | | CUC, CUG, CUU |
| M | Met | Methionine | AUG |
| N | Asn | Asparagine | AAC, AAU |
| P | Pro | Proline | CCA, CCC, CCG, |
| | | | CCU |
| Q | Gln | Glutamine | CAA, CAG |
| R | Arg | Arginine | AGA, AGG, CGA, |
| | | | CGC, CGG, CGU |
| S | Ser | Serine | AGC, AGU, UCA, |
| | | | UCC, UCG, UCU |
| T | Thr | Threonine | ACA, ACC, ACG, |
| | | | ACU |
| V | Val | Valine | GUA, GUC, GUG, |
| | | | GUU |
| W | Trp | Tryptophan | UGG |
| Y | Tyr | Tyrosine | UAC, UAU |

As noted above, the invention provides for preserved solutions and formulations containing a preservative as well as multi-use preserved formulations suitable for pharmaceutical or veterinary use, comprising FSH or FSH variant in a pharmaceutically acceptable formulation. Preserved formulations contain the preservative benzyl alcohol in an aqueous diluent.

As noted above, the invention provides an article of manufacture, comprising packaging material and a vial comprising a solution of FSH or a FSH variant with the prescribed buffers and/or preservatives, optionally in an aqueous diluent, wherein said packaging material comprises a label which indicates that such solution may be held over a period of twenty-four hours or greater.

The FSH or a FSH variant used in accordance with the present invention may be produced by recombinant means, including from mammalian cell or transgenic preparations, or may be purified from other biological sources, such as from urinary sources. Acceptable methodologies include those described in Hakola, K. Molecular and Cellular Endocrinology, 127:59-69, 1997; Keene, et al., J. Biol. Chem., 264:4769-4775, 1989; Cerpa-Poljak, et al., Endocrinology, 132:351-356, 1993; Dias, et al., J. Biol. Chem., 269:25289-25294, 1994; Flack, et al., J. Biol. Chem., 269:14015-14020, 1994; and Valove, et al., Endocrinology, 135:2657-2661, 1994, and U.S. Patent 3,119,740.

The method by which the proteins are provided for the formulations of this invention is not particularly relevant. Preferably FSH is a heterodimer comprising one alpha subunit and one beta subunit, respectfully, as provide in SEQ ID NOS 5 and 6, or a FSH variant heterodimer comprising one alpha subunit and one beta subunit, respectfully, as given in SEQ ID NOS: 5 and 11; 5 and 12; and 5 and 13. Suitable FSH or a FSH variant species within the present invention include, but are not limited to, at least one known alpha subunit sequence and at least one known beta subunit (see sequence listing for known alpha and beta subunits and as otherwise known in the art).

Non-limiting examples of FSH or a FSH variant, include but are not limited to:
(a) α-subunit: (SEQ ID NO:1) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
 β -subunit: (SEQ ID NO:2) 
RSCELTNITITVEKEECGFCISINTTWCAGYCYTRDLVYRDPARPNIQKTCTFKEL
VYETVKVPGCAHHADSLYTYPVATECHCSKCDSDSTDCTVRGLGPSYCSFREIKE
(b) :α-subunit: (SEQ ID NO:3) 
FPDGEFTTQDCPECKLRENKYFFKLGVPIYQCKGCCFSRAYPTPARSRKTMLVPKN
ITSESTCCVAKAFIRVTVMGNIKLENHTQCYCSTCYHHKI
 β -subunit: (SEQ ID NO:4) 
NSCELTNITIAVEKEGCGFCITINTTWCAGYCYTRDLVYKDPARPNIQKTCTFKEL
VYETVKVPGCAHHADSLYTYPVATACHCGKCNSDSTDCTVRGLGPSYCSFGDMKE
(c) :α-subunit: (SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -subunit: (SEQ ID NO:6) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMKE
(d) :α-subunit: (SEQ ID NO:7) 
FPDGEFTMQGCPECKLKENKYFSKLGAPIYQCMGCCFSRAYPTPARSKKTMLVPKN
ITSEATCCVAKAFTKATVMGNARVENHTECHCSTCYYHKS
 β -subunit: (SEQ ID NO:8) 
NSCELTNITITVEKEECNFCISINTTWCAGYCYTRDLVYKDPARPNIQKTCTFKEL
VYETVKVPGCAHHADSLYTYPVATECHCGKCDSDSTDCTVRGLGPSYCSFSEMKE
(e) :α-subunit: (SEQ ID NO:9) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
 β -subunit: (SEQ ID NO:10) 
RSCELTNITITVEKEECSFCISINTTWCAGYCYTRDLVYKDPARPNIQKACTFKEL
VYETVKVPGCAHHADSLYTYPVATECHCGKCDRDSTDCTVRGLGPSYCSFSDIRE
(f) :α-subunit: (SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -subunit: (SEQ ID NO:11) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGE
(g) :α-subunit: (SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -subunit: (SEQ ID NO:12) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEM
(h) :α-subunit: (SEQ ID NO:5) 
APDVQDCPECTLQENPFFSQPGAP ILQCMGCCFSRAYPTPLRSKKTMLVQKNVTSE
STCCVAKSYNRVTVMGGFKVENHTACHCSTCYYHKS
 β -subunit: (SEQ ID NO:13) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMK
The range of protein hormone in the product of the present invention includes amounts yielding upon reconstitution, if in a wet/dry system, concentrations from about 1.0 µg/ml to about 50 mg/ml, although lower and higher concentrations are operable and are dependent on the intended delivery vehicle, e.g., solution formulations will differ from transdermal patch, pulmonary, transmucosal, or osmotic or micro pump methods. The hormone concentrations are preferably about 5.0 µg/ml to 2 mg/ml and most preferably about 5.0 µg/ml, or 10 µg/ml, or 50 µg/ml to about 200 µg/ml.

The aqueous diluent further comprises a pharmaceutically acceptable preservative: benzyl alcohol. The concentration of preservative used in the formulation is a concentration sufficient to yield an anti-microbial effect, and is readily determined by the skilled artisan. Surprisingly, the preservative used in the presently claimed formulations does not adversely affect the biological activity of FSH or a FSH variant and allow multi-use administration.

Other excipients, e.g. isotonicity agents, buffers, antioxidants, preservative enhancers, may be optionally and preferably added to the diluent. An isotonicity agent, such as glycerin, is commonly used at known concentrations. The concentration of glycerin is generally about 16 mg/ml. A physiologically tolerated buffer is preferably added to provide improved pH control. The formulations may cover a wide range of pHs, from pH 6.8 to pH 9 and a preferred range to about 8.0. Preferably the formulations of the present invention have pH between about 6.8 and about 7.8.
Preferred buffers include phosphate buffers, most preferably sodium phosphate, particularly phosphate buffered saline (PBS).

Other additives, such as a pharmaceutically acceptable solubilizers like Tween 20 (polyoxyethylene (20) sorbitan monolaurate), Tween 40 (polyoxyethylene (20) sorbitan monopalmitate), Tween 80 (polyoxyethylene (20) sorbitan monooleate), Pluronic F68 (polyoxyethylene polyoxypropylene block copolymers), and PEG (polyethylene glycol) or non-ionic surfactants such as polysorbate 20 or 80 or poloxamer 184 or 188, Pluronic® polyls, other block co-polymers, and chelators such as EDTA and EGTA may optionally be added to the formulations or compositions to reduce aggregation. These additives are particularly useful if a pump or plastic container is used to administer the formulation. The presence of pharmaceutically acceptable surfactant mitigates the propensity for the protein to aggregate. The present claimed formulations are surprisingly stable. Prior to the present invention, the preparation of preserved, multi-use formulations of FSH was believed to be impossible due to instability. Applicants have discovered that the claimed formulations may be safely stored at temperatures of from about 2 to about 40°C and retain the biologically activity of the protein for extended periods of time, exceeding 2 months and as further demonstrated.

The formulations of the present invention can be prepared by a process which comprises mixing FSH or a FSH variant and benzyl alcohol in an aqueous diluent. Mixing the FSH or a FSH variant and preservative in an aqueous diluent is carried out using conventional dissolution and mixing procedures. To prepare a suitable formulation, for example, a measured amount of FSH or a FSH variant in buffered solution is combined with the desired preservative in a buffered solution in quantities sufficient to provide the protein and preservative at the desired concentrations. Variations of this process would be recognized by one of ordinary skill in the art. For example, the order the components are added, whether additional additives are used, the temperature and pH at which the formulation is prepared, are all factors that may be optimized for the concentration and means of administration used.

The claimed formulations may be provided to patients as clear solutions. The single solution vial may be reused multiple times and may suffice for a single or multiple cycles of patient treatment and thus provides a more convenient treatment regimen than currently available.

The present claimed articles of manufacture are surprisingly useful for administration over a period of twenty-four hours or greater. Prior to the present invention, such products were only suitable and approved for immediate use. The patient was instructed to discard unused material leading to waste and expense. Accordingly, the presently claimed articles of manufacture offer significant advantages to the patient. Applicants have discovered that the claimed formulations may be safely stored at temperatures of from about 2 to about 40°C and retain the biologically activity of the protein for extended periods of time, exceeding 2 months; thus, allowing a package label indicating that the solution may be held and/or used over a period of 24, 36, 48, 72, or 96 hours or greater. If preserved diluent is used, such label may include use up to one, one and a half, to two years.

The solutions of FSH or a FSH variant in the invention can be prepared by a process which comprises mixing FSH or a FSH variant in an aqueous diluent. Mixing is carried out using conventional dissolution and mixing procedures. To prepare a suitable diluent, for example, a measured amount of FSH or a FSH variant in water or buffer is combined in quantities sufficient to provide the protein and optionally a preservative or buffer at the desired concentrations. Variations of this process would be recognized by one of ordinary skill in the art. For example, the order the components are added, whether additional additives are used, the temperature and pH at which the formulation is prepared, are all factors that may be optimized for the concentration and means of administration used.

The claimed products may be provided to patients as clear solutions. The single solution vial may be reused multiple times and may suffice for a single or multiple cycles of patient treatment and thus provides a more convenient treatment regimen than currently available.

The claimed products may be provided indirectly to patients by providing to pharmacies, clinics, or other such institutions and facilities, clear solutions. The clear solution in this case may be up to one liter or even larger in size, providing a large reservoir from which smaller portions of the FSH or a FSH variant solution may be retrieved one or multiple times for transfer into smaller vials and provided by the pharmacy or clinic to their customers and/or patients. The clear solution provided by the pharmacy or clinic to their customers and patients may suffice for single or multiple cycles of patient treatment and thus provides a more convenient treatment regimen than currently available.

Recognized devices comprising these single vial systems include those pen-injector devices for delivery of a solution such as Humaject®, NovoPen®, B-D®Pen, AutoPen®, and OptiPen®.

The products presently claimed include packaging material. The packaging material provides, in addition to the information required by the regulatory agencies, the conditions under which the product may be used. For the single vial, solution product, the label indicates that such solution may be used over a period of twenty-four hours or greater. The presently claimed products are useful for human pharmaceutical product use.
The claimed stable or preserved formulations may be provided to patients as clear solutions. The single solution vial may be reused multiple times and may suffice for a single or multiple cycles of patient treatment and thus provides a more convenient treatment regimen than currently available.

FSH or a FSH variant in either the stable or preserved formulations or solutions described herein, may be administered to a patient in accordance with the present invention via a variety of delivery methods including SC or IM injection; transdermal, pulmonary, transmucosal, implant, osmotic pump, cartridge, micro pump, oral, or other means appreciated by the skilled artisan, as well-known in the art.

The following examples are provided merely to further illustrate the preparation of the formulations and compositions of the invention. The scope of the invention shall not be construed as merely consisting of the following examples.

### Example 1

### Cloning and Expression of FSH or a FSH variant, in Mammalian Cells

A typical mammalian expression vector contains at least one promoter element, which mediates the initiation of transcription of mRNA, the polypeptide coding sequence, and signals required for the termination of transcription and polyadenylation of the transcript. However, because functional FSH or FSH variants contain both an alpha and beta subunit, means to express both subunits are required, either by expressing both subunits from single vector containing a promoter element for each subunit, or by using two vectors: a first vector containing a promoter to express the first subunit and a second vector that has a promoter to express the second subunit.

Additionally, each mammalian expression vector have elements that may be present on one or more vectors include enhancers, Kozak sequences and intervening sequences flanked by donor and acceptor sites for RNA splicing.

Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in providing FSH or FSH variants subunits include, for example, vectors such as pIRESlneo, pRetro-Off, pRetro-On, PLXSN, or pLNCX (Clonetech Labs, Palo Alto, CA), pcDNA3.1 (+/-), pcDNA/Zeo (+/-) or pcDNA3.1/Hygro (+/-) (Invitrogen), PSVL and PMSG (Pharmacia, Uppsala, Sweden), pRSVcat (ATCC 37152), pSV2dhfr (ATCC 37146) and pBC12MI (ATCC 67109). Mammalian host cells that could be used include human Hela 293, H9 and Jurkat cells, mouse NIH3T3 and C127 cells, Cos 1, Cos 7 and CV 1, quail QC1-3 cells, mouse L cells and Chinese hamster ovary (CHO) cells.

Alternatively, the desired DNA sequences for the alpha and beta subunit can be expressed in stable cell lines that contain the DNA sequences for expressing each subunit once integrated into a chromosome(s). The co-transfection with a selectable marker such as dhfr, gpt, neomycin, or hygromycin allows the identification and isolation of the transfected cells as known in the art.

The transfected DNA sequences for the subunits can also be amplified to express large amounts of the encoded polypeptide. The DHFR (dihydrofolate reductase) marker is useful to develop cell lines that carry several hundred or even several thousand copies of the DNA sequences of interest. Another useful selection marker is the enzyme glutamine synthase (GS) (Murphy, et al., Biochem. J. 227:277-279 (1991); Bebbington, et al., Bio/Technology 10:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins and polypeptides.

The expression vectors pC1 and pC4 contain the strong promoter (LTR) of the Rous Sarcoma Virus (Cullen, et al., Molec. Cell. Biol. 5:438-447 (1985)) plus a fragment of the CMV-enhancer (Boshart, et al., Cell 41:521-530 (1985)). Multiple cloning sites, e.g., with the restriction enzyme cleavage sites BamHI, XbaI and Asp718, facilitate the cloning of the DNA sequences for the alpha and beta subunits of interest. The vectors contain in addition the 3' intron, the polyadenylation and termination signal of the rat preproinsulin gene.

### Example 2

### Cloning and Expression in COS or CHO Cells

An expression plasmid for FSH or a FSH variant is made by cloning a cDNA encoding FSH or a FSH variant subunits into the expression vector pcDNAI/Amp or pcDNAIII (which can be obtained from Invitrogen, Inc.). As previously mentioned, each subunit requires expression to produce a function heterodimer, either from independent introduction of separate vectors into the host cell or by engineering a single vector to express both alpha and beta subunits.

The expression vector(s) pcDNAI/amp contains: (1) an E. coli origin of replication effective for propagation in E. coli and other prokaryotic cells; (2) an ampicillin resistance gene for selection of plasmid-containing prokaryotic cells; (3) an SV40 origin of replication for propagation in eucaryotic cells; (4) a CMV promoter, a polylinker, an SV40 intron; (5) several codons encoding a hemagglutinin fragment (i.e., an "HA" tag to facilitate purification) or HIS tag (see, e.g., Ausubel, supra) followed by a termination codon and polyadenylation signal arranged so that a cDNA can be conveniently placed under expression control of the CMV promoter and operably linked to the SV40 intron and the polyadenylation signal by means of restriction sites in the polylinker. The HA tag corresponds to an epitope derived from the influenza hemagglutinin polypeptide described by Wilson, et al., Cell 37:767-778 (1984). The fusion of the HA tag to the target polypeptide, either the alpha or beta subunit, allows easy detection and recovery of the recombinant polypeptide with an antibody that recognizes the HA epitope. pcDNAIII contains, in addition, the selectable neomycin marker.

A DNA fragment encoding the alpha and beta subunit of FSH or a FSH variant, is separately cloned into the polylinker region of the vector so that recombinant polypeptide expression is directed by the CMV promoter. Insertion into the vector is optionally with or without the HA epitope. The plasmid construction strategy is as follows. The FSH or a FSH variant, cDNA of the deposited clone for each subunit is amplified using primers that contain convenient restriction sites.

The PCR amplified DNA fragment for each subunit and the vector, pcDNAI/Amp, are digested with suitable restriction enzyme(s) and then each subunit is ligated to digested vector. Each ligation mixture is transformed into E. coli strain SURE (available from Stratagene Cloning Systems, 11099 North Torrey Pines Road, La Jolla, CA 92037), and the transformed culture is plated on ampicillin media plates which then are incubated to allow growth of ampicillin resistant colonies. Plasmid DNA for each subunit is isolated from resistant colonies and examined by restriction analysis or other means for the presence of the FSH or a FSH variant encoding fragment.

For expression of recombinant FSH or a FSH variant, COS cells are co-transfected with an expression vector for each subunit, as described above, using DEAE-DEXTRAN, as described, for instance, in Sambrook, et al., Molecular Cloning: a Laboratory Manual, Cold Spring Laboratory Press, Cold Spring Harbor, New York (1989). Cells are incubated under conditions for expression of FSH or a FSH variant, by each vector.

It is expected that expression of the FSH HA or FSH variant HA fusion polypeptide is detected by radiolabeling and immunoprecipitation, using methods described in, for example Harlow, et al., Antibodies: A Laboratory Manual, 2nd Ed., Cold Spring Harbor Laboratory Press, Cold Spring Harbor, New York (1988). To this end, two days after transfection, the cells are labeled by incubation in media containing 35S-cysteine for 8 hours. The cells and the media are collected, and the cells are washed and lysed with detergent-containing RIPA buffer: 150 mM NaCl, 1% NP-40, 0.1% SDS, 0.5% DOC, 50 mM TRIS, pH 7.5, as described by Wilson, et al. cited above. Proteins are precipitated from the cell lysate and from the culture media using an HA-specific monoclonal antibody. The precipitated protein is then are analyzed by SDS-PAGE and autoradiography. An expression product of the expected size is seen in the cell lysate, which is not seen in negative controls.

The vector pC4 is used for the expression of each subunit of FSH or a FSH variant. Alternatively, one skilled in the art would be able to adapt pC4 to express both alpha and beta subunits from a single vector. Plasmid pC4 is a derivative of the plasmid pSV2-dhfr (ATCC Accession No. 37146). The plasmid contains the mouse DHFR gene under control of the SV40 early promoter. Chinese hamster ovary- or other cells lacking dihydrofolate activity that are co-transfected with alpha and beta subunit plasmids can be selected by growing the cells in a selective medium (alpha minus MEM, Life Technologies) supplemented with the chemotherapeutic agent methotrexate. The amplification of the DHFR genes in cells resistant to methotrexate (MTX) has been well documented (see, e.g., F. W. Alt, et al., J. Biol. Chem. 253:1357-1370 (1978); J. L. Hamlin and C. Ma, Biochem. et Biophys. Acta 1097:107-143 (1990); and M. J. Page and M. A. Sydenham, Biotechnology 9:64-68 (1991)). Cells grown in increasing concentrations of MTX develop resistance to the drug by overproducing the target enzyme, DHFR, as a result of amplification of the DHFR gene. If DNA sequences are linked to the DHFR gene, it is usually co-amplified and over-expressed. It is known in the art that this approach can be used to develop cell lines carrying more than 1,000 copies of the amplified gene(s). Subsequently, when the methotrexate is withdrawn, cell lines are obtained which contain the amplified DNA sequences integrated into one or more chromosome(s) of the host cell.

Plasmid pC4 contains for expressing the alpha and beta subunit DNA sequences of interest behind the strong promoter of the long terminal repeat (LTR) of the Rous Sarcoma Virus (Cullen, et al., Molec. Cell. Biol. 5:438-447 (1985)) which additionally contains a fragment isolated from the enhancer of the immediate early gene of human cytomegalovirus (CMV) (Boshart, et al., Cell 41:521-530 (1985)). Downstream of the promoter are BamHI, XbaI, and Asp718 restriction enzyme cleavage sites that allow integration of the DNA sequences. Behind these cloning sites the plasmid contains the 3' intron and polyadenylation site of the rat preproinsulin gene. Other high efficiency promoters can also be used for the expression, e.g., the human b-actin promoter, the SV40 early or late promoters or the long terminal repeats from other retroviruses, e.g., HIV and HTLVI. Clontech's Tet-Off and Tet-On gene expression systems and similar systems can be used to express the FSH or a FSH variant, in a regulated way in mammalian cells (M. Gossen, and H. Bujard, Proc. Natl. Acad. Sci. USA 89: 5547-5551 (1992)). For the polyadenylation of the mRNA other signals, e.g., from the human growth hormone or globin genes can be used as well. Stable cell lines carrying the DNA sequences of the alpha and beta subunit integrated into the chromosomes can also be selected upon co-transfection with a selectable marker such as gpt, G418 or hygromycin. It is advantageous to use more than one selectable marker in the beginning, e.g., G418 plus methotrexate.

The plasmid pC4 is digested with restriction enzymes and then dephosphorylated using calf intestinal phosphatase by procedures known in the art. The vector is then isolated from a 1% agarose gel.

The DNA sequence encoding the complete FSH or a FSH variant, for each subunit is amplified using PCR oligonucleotide primers corresponding to the 5' and 3' sequences of the gene. Non-limiting examples include 5' and 3' primers having nucleotides corresponding or complementary to a portion of the coding sequence of each subunit for an FSH or a FSH variant according to known methods in the art.

The amplified fragment(s) are digested with suitable endonucleases and then purified again on a 1% agarose gel. The isolated fragments for each subunit and the dephosphorylated vector are then separately ligated with T4 DNA ligase. E. coli HB101 or XL-1 Blue cells are then are separately transformed and bacteria are identified that contain the fragment (or fragments if the vector is adapted for expressing both alpha and beta subunits) inserted into plasmid pC4 using, for instance, restriction enzyme analysis.

Chinese hamster ovary (CHO) cells lacking an active DHFR gene are used for transfection. 5 µg of the expression plasmid(s) pC4 is cotransfected with 0.5 µg of the plasmid pSV2-neo using lipofectin. The plasmid pSV2neo contains a dominant selectable marker, the neo gene from Tn5 encoding an enzyme that confers resistance to a group of antibiotics including G418. The cells are seeded in alpha minus MEM supplemented with 1 µg/ml G418. After 2 days, the cells are trypsinized and seeded in hybridoma cloning plates (Greiner, Germany) in alpha minus MEM supplemented with 10, 25, or 50 ng/ml of methotrexate plus 1 µg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 mM, 2 mM, 5 mM, 10 mM, 20 mM). The same procedure is repeated until clones are obtained which grow at a concentration of 100 - 200 mM. Expression of the desired product is analyzed, for instance, by SDS-PAGE and Western blot or by reverse phase HPLC analysis.

It will be clear that the invention can be practiced otherwise than as particularly described in the foregoing description and examples.

Numerous modifications and variations of the present methodologies is known in the art to expressing recombinant proteins, including those as described in Keene J.L., et al., J. Biol. Chem., 264:4769-4752, 1989; Loumaye E., et al., Human Reprod. Update, 1:188-1999, 1995; Olijve W., et al., Mol. Hum. Reprod., 2:361-369, 1996, as well as other recombinant techniques known and used for other gonadotropins.

### Example 3

### Expression in AV12 Cells

An expression cassett vector pGTH was used for expression of the alpha subunit in AV12. A number of published articles describe the use of AV12-664 and/or AV12-RGT18 cells (see Grinnell, B.W. et al., Blood 76(12) :2546-54, 1990; Burck, P.J. et al., J. Biol. Chem. 265(9):5170-7, 1990; Parkinson, J.F. et al., J. of Biol. Chem. 265(21):12602-10, 1990; Grinnell, B.W. et al., J. Biol. Chem 266(15):9778-85, 1991; Wery, J.P. et al., Nature 352(6330):79-82, 1991; Berg, D.T. et al., Biotechniques 14:972-9, 1993; Gerlitz, B. et al. Biochemical Journal 295 (1):131-40, 1993; Kursar, J.D. et al. Molecular Pharmacology 46(2):227-34, 1994; Desai, M.A. et al. Molecular Pharmacology 48(4):648-57, 1995; Obesity Research 3 Suppl 4:4441S-4447S, 1995; Desai, M.A. et al. British Journal of Pharmacology 118(6):1558-64, 1996; Kumar, A. et al. Cancer Research 56(23):5397-402, 1996; Boggs, L.N. et al. J. of Neurochemistry 57(3):1324-7, 1996; Lucaites, V.L et al. Life Sciences 59(13):1081-95, 1996; Schoepp, D.D. et al. Neuropharmacology 35(12):1661-72, 1996; Kumar, A. et al. Cancer Research 57(15):3111-4, 1997; Urology 49(3):487-93, 1997; Wainscott, D.B. et al. Naunyn-Schmiedebergs Archives of Pharacology 357(1):17-24, 1998; Wu, S. et al. Brain Research - Molecular Brain Research 53(1-2):88-97, 1998. Similarly, publications have described use of the plasmid pGTH (see Grinnell, et al. J. Biol. Chem. 266(15):9778-85, 1991) and the plasmid pGTD (see Biolchemical Journal 295 (Pt1): 131-40, 1993).

Briefly, pGTH contains sequentially several elements: the SV40 early promoter.ori, E.coli hygromicin resistance, SV40 small "t" antigen splice site/poly-A site, pBR322 cloning remnant, BK virus (strain P2) cloning remnant, Poly-CA₂₀/GT₂₀ element (synthetic oligonucleotide), BK virus (strain P2) enhancer, AD2 major late promoter/spliced tripartite leader, Bcll insertion site for the FSH alpha subunit coding sequence (including stop codon), SV40 small "t" antigen splice site/poly-A site; and pBR322 ampicillin resistance/ori.

The plasmid construct pGTH-alpha was generated to express the encoded the human alpha subunit sequence (SEQ ID:5) by cloning a 362-bp BclI FSH cDNA fragment into the unique BclI site of the vector (see sequence - SEQ ID:19 or 14). The FSH alpha cDNA fragment DNA was generated by PCR amplification using the shuttle plasmid pLGD637 as template (pLGD637 contains a synthetic/oligonuclotide-assembled FSH alpha cDNA sequence). The integrity of BclI insert was confired by sequencing followed by comparison to the GenPept database (Accession Number 31869).

An expression cassett vector pGTD was used to express the human beta subunit FSH variant sequence (SEQ ID:11). pGTD contains several elements for expression in AV12 cells. pGTD contains sequentially the BK virus (strain P2) cloning remnant, Poly-CA₂₀/GT₂₀ element (synthetic oligonucleotide), BK virus (strain P2) enhancer, AD2 major late promoter/spliced tripartite leader, Bcll insertion site for the FSH variant beta subunit coding sequence (including stop codon), SV40 small "t" antigen splice site/poly-A site; SV40 early promoter.ori, Murine dihydrofolate reductase cDNA, SV40 small "t" antigen splice site/poly-A site, and pBR322 ampicillin resistance/ori.

The plasmid construct pGTD-bCD3 was generated by cloning a 393-bp BclI FSH beta-bCD3 cDNA fragment into the unique BclI site of the pGTD vector (see SEQ ID:20 or 15). The FSH beta-CD3 cDNA fragment DNA was generated by PCR amplification, using the shuttle plasmid pLGD638 as template (pLGD638 contains a synthetic/oligonucleotide-assembled FSH beta cDNA sequence). The integrity of the construct was confirmed by sequencing and compared with the human beta subunit sequence deposited in the GenPept database (Accession Number 476441).

In brief, the pGTH-alpha and pGTD-bCD3 plasmids were linearized, repurified, and then co-transfected into adherent AV23-RGT18 cells. Following selection with medium containing 0.25uM methotrexate and 100 µg/ml hygromycin-B, along with 200 µg/ml G418 to maintain the glutamate transporter genotype of the AV12-RGT18 cells, individual stable clones were isolated either manually or via flow-assisted cell sorting. Highest producing clones were identified by analysis of conditioned medium with a commercial FSH Elisa kit. Several clones were adapted to serum-free suspension and further amplified to obtain isolatable quantities of the FSH variant heterodimer.

### Example 4

### Expression In CHO-K1 Cells

A CHO-K1 cell line (LONZA Biologics plc.) was developed to produce a FSH variant heterodimer composed of the alpha subunit of SEQ ID NO:5 and the beta subunit of SEQ ID NO:11.

The expression vector cassette contains the encoding DNA for alpha subunit, SEQ ID NO:5, and the encoding DNA for the beta subunit, SEQ ID NO:11. are controlled by two different promoters: CMV for the beta subunit and EF1 Alpha for the alpha subunit. Each alph and beta subunit sequence uses the Bovine Growth Hormone polyA tail. Additionally the vector contains the Glutamine Synthetase gene, controlled by the SV40 Late Promoter and containing the SV40 polyA tail, is used as the selectable marker. This vector was used to transfect the CHO-K1 cells.

The cell line was grown in GibcoBRL's CD CHO media under selective pressure of L-methionine sulfoximine. ELISA assays were used to identify master wells expressing FSH variant. Several master wells were subjected to cloning and amplification procedures. These experiments led to the clonal cell line 2B6.1C3.25 which had suitable titers. Expression studies conducted in small scale shake flasks (20-60 ml) have shown that this line expresses FSH variant at 30 mg/L after 7 to 8 days.

### Example 5

### Purification of FSH variants from CHO or AV-12 Cells

Purification of FSH variant heterodimer comprised of an alpha subunit of SEQ ID NO:5 and a beta subunit of SEQ ID:11 can be accomplished by a number of methods described and known in the art from monolayer or suspension cultures of either CHO-K1 or AV12 cell lines or other production lines suitably available. One method for isolating the disclosed FSH variant from the culture containing medium is subjecting the culture medium to cation exchange chromatography, dye affinity chromatography and gel filtration chromatography to purify the protein. In the case of suspension cultures, which may contain detergents, additional purification steps may be needed such as a Q-Sepharose step. The chromatographic steps can be further added to or optimized for pH, conductivity, buffer composition and running conditions (column dimensions, flow rates, etc). Purity and yield can be analyzed by SDS-PAGE gels (both Coomassie staining and Western blotting), ELISA assays, exclusion chromatography and protein concentration by UV absorbance at 277nm or other known techniques.

Purification and chromatographic fractionation can be achieved by following the further details for each isolation step given below. For monolayer cultures, the conditioned medium is concentrated and diafiltered prior to application to the cation exchange column. The Q-Sepharose batching step can be included for suspension cultures to remove detergents that may be present.

### 1. Concentration

Typically if 0.02 to 0.04% Pluronic F68 is used for AV12 suspension cultures, whereas there is 0.1 to 0.18% in the media used for CHO suspension cultures. The conditioned medium, clarified by the cell culture group using centrifugation and filtration through a cheese cloth, is concentrated using a tangential flow filtration system ProFlux (ProFlux M12 from Millipore) with a S3YM10 spiral cartridge (Amicon #540633). Depending on the amount of Pluronic F68 that was used, the medium is concentrated 4 to 10-fold so that there is 0.2-0.4% Pluronic F68 in the concentrated medium. The final volume of material after concentration is usually 2-3L starting from 8L for CHO-K1 and 24L for AV12 cultures.

### 2. Batching with Q-Sepharose

For each liter of starting conditioned medium before concentration, 50 ml of Fast Flow Q-Sepharose resin (Pharmacia 17-0510-01, pre-equilibrated with 20 mM Tris, pH 7.4) and sufficient NaCl to give a final conductivity of 200 mM (∼20 mS/cm) are added to the concentrated medium and stirred gently overnight at 4°C

### 3. Diafiltration

After overnight batching of the concentrated medium with Q-Sepharose resin, the resin is allowed to settle, the supernatant is decanted out and filtered using a CUNO system with a Zeta Plus 30-SP Filter (#B0406-30SP from Sun Source Fauver) and a Masterflex pump at a flow rate of 170 ml/min. The medium is then further concentrated to about 800 ml and diafiltered using 5-6 volumes of 20 mM Tris pH 7.4 in the Proflux system. At this point, the conductivity is - 2 mS/cm. The pH is adjusted to 5.0 with 1N HCl and the solution is again filtered using a fresh Zeta Plus 30-SP filter in the CUNO system and immediately loaded onto the Cation Exchange column.

### 4. Cation Exchange Chromatography (CEX)

Column: Pharmacia SP-Sepharose Fast Flow (17-0729-01) is used to pack a 50 ml column for ∼100-200 mg of FSH (total protein - 500-600 mg). Buffers are: A: 20 mM sodium phosphate, pH 5.0; and B: 20 mM sodium phosphate, pH 5.0, 1M NaCl

The sample is adjusted to pH 5.0, clarified by filtration and immediately loaded onto the column and run at 5 ml/min with 4 Column Volumes before starting a gradient of 0% to 50% B over 15 Column Volumes. 3 min fractions are collected (15 ml). Fractions are collected into 400 ml of 1 M Tris, pH 8.0.

Coomassie-stained SDS PAGE gels are used to choose FSH-containing fractions to pool (typically the pool size is 200 to 250 ml for a 50 ml column). This pool is dialyzed against 20 mM Tris, pH 7.4 overnight at 4°C to drop conductivity to < 3 mS/cm.

### 5. Dye Affinity Chromatography (DAC)

Column: Mimetic Blue Dye 1 A6XL, 0090-0500 from Prometic Biosciences Inc. is used in a 50 ml column for ∼40 mg of FSH). Buffers are: A: 25 mM phosphate, pH 6.5 (conductivity is 4.5 to 5 mS/cm); B: 25 mM phosphate, 150 mM KCl, pH 6.5; C: 25 mM phosphate, 1M KCl, pH 8.0

After dialysis of CEX pool, the pH is adjusted to 6.5, and loaded onto the DAC column at 3 ml/min. A gradient from 100%A to 50%A; 50% B is applied for 4 Column Volumes, then eluted with 100% Buffer C for 5 Column Volume, collecting Fractions of 3 min (9 ml).

Coomassie-stained SDS-PAGE gels are used to choose FSH-containing fractions to pool. Typically, the pool size is 90 to 100 ml for a 50 ml column. The pool is concentrated to 4 ml using Millipore Ultrafree centrifuge devices (UFV2BCC40, 5000 MWCO, spun at 2000 rpm) and loaded onto a Gel Filtration column.

### 6. Gel Filtration

Column: BioPilot Superdex 75 Prep Grade 35/600 column is used for 50 to 100 mg of FSH. Buffers are: 1x PBS (made from GIBCO 10x PBS, #70011) plus 100 mM NaCl. Final composition of the buffer is 1 mM monobasic potassium phosphate, 3 mM dibasic sodium phosphate, 253 mM sodium chloride, pH 7.4.

The column is loaded with 4 ml of FSH from DAC step in 1x PBS as described above at a flow rate: 3 ml/min collecting 1 min (3 ml) fractions.

The purity of FSH after this step is usually >95% by Coomassie and silver-stained gels.

### EXAMPLE 6

### Effect Of Preservatives On Physical Stability Of FSH

Since preservatives tend to denature or destabilize protein or induce aggregation (Brange, J. and Langkjar, L., Acta Pharm. Nord, 4, 149-158, 1992; Maa YF and Hsu C, International Journal of Pharmaceutics, 140, 155-168, 1996), the physical stability of uFSH (uFSH - Vitro Diagnostics - Human Urofollitropin) in the presence of different preservatives was examined using the dynamic light scattering technique. All measurements were obtained with a system consisting of a Lexel 95 Argon Laser (488 nm), a Brookhaven Instruments model BI-200SM goniometer, and a BI9000AT autocorrelator. Data parameters consist of: initial photon counts adjusted to 100,000 counts/sec, 30 second duration, 31 dust cutoff value, and a 90° scattering angle.

Preservatives were added to a 1.5 ml solution of 1 mg/ml urinary follicle stimulatin hormone (uFSH - Vitro Diagnostics - Human Urofollitropin) which had been dialyzed against 1 x PBS overnight at pH 7.4. The concentration of preservative was selected to be the concentration generally known to provide adequate anti-microbial activity. In a laminar flow hood, this sample was filtered through a 0.1 µm Anotop-Plus filter (10mm) into a 12 mm DLS test tube. The sample was placed in the DLS holder which had been equilibrated at 37°C. The auto-correlation function was determined every 15 minutes for 24 hours and analyzed to yield the hydrodynamic parameter. This measurement demonstrated that more than 99% of the protein molecules had an average diameter of about 5.7 nm. A small population (<1%) had an average diameter of about 200 nm. The presence of the preservatives did not change the size distribution of the molecule appreciably after 24 hour at 37°C. The representative data at 24 hr. was then analyzed using NNLS (Non Negative Linear Squares) program as shown in Table I. More than 99% of the molecules were in particles with an average diameter of about 5.7 nm. Using an empirical equation relating crystallographically determined hydrodynamic radius with molecular weight (Squire, P.G. and Himmel, M.E., in Arch. Bioch. Biophys., 196, pp. 165-177, 1979) this average DLS particle size corresponds to about 36,000 daltons, which is consistent with the molecular weight of the uFSH heterodimer. The remaining small population of particles (<1%) had an average diameter of about 200 nm. These data show, in Table VI that the preservatives studied did not significantly aggregate uFSH under the conditions tested.

**Table VI.**

| Size distribution of uFSH in formulations containing different preservatives. | | | |
|---|---|---|---|
| Preservative | Preservative Concentration (mg/ml) | Small Particles (∼ 5.7 nm) | Large Particles (∼ 200 nm) |
| None | 0 | >99 % | < 1 % |
| *m*-cresol | 3.5 | >99 % | < 1 % |
| Phenol | 3.5 | >99 % | < 1 % |
| Benzyl alcohol | 10 | >99 % | < 1 % |
| Methylparaben | 1 | >99 % | < 1 % |
| Chlorocresol | 2 | >99 % | < 1 % |

### EXAMPLE 7

### Thermal Denaturation Studies on uFSH Formulations

The thermal unfolding transition for uFSH (uFSH - Vitro Diagnostics - Human Urofollitropin) as a function of solvent conditions was monitored by differential scanning calorimetry (DSC). Experiments were carried out on a VP-DSC MicroCalorimeter (MicroCal inc., Northampton, MA; Plotnik, V.V., et. al., Anal. Biochem., 250:237-244, 1997) using VPViewer software for data acquisition and Origin DSC software for data analysis. The matched sample cell and reference cell were lollipop-shaped, fabricated from tantalum, with a working volume of 0.5 ml. Approximately 1 mg/ml uFSH samples were dialyzed against appropriate buffer overnight and concentration of the protein in the sample was determined by UV spectroscopy. The proteins were then diluted to 0.4 - 0.5 mg/ml for the DSC experiments. The dialysate was used as reference solution. Sample and reference solutions were degassed for 5 minutes before loading into the cells with a 2.5 ml needle through a filling funnel. Pressure was kept at about 30 psi with the pressure cap. For all measurements in this study, the instrument was run overnight with buffers in both the reference cell and sample cell to establish thermal history prior to sample runs. The data was analyzed with Origin DSC software using a two-state model (Sturtevant, J.M., Annu. Rev. Phys. Chem., 38:463-488, 1987). The midpoint of the transition temperature (Tₘ ) at different solution conditions is summarized in Table VII. The protein undergoes a very cooperative transition with a Tₘ of 77.3 °C in PBS buffer at pH 7.4. Thermal denaturation is irreversible on the time scale of the measurements as shown by the absence of transition in the second scan immediately following the first scan. However, the dissociated subunits stay as monomers in solution and these monomers can then reassociate to form biologically active dimer in the course of the days (data not shown). The effects of the addition of preservatives, *m*-cresol, phenol, benzyl alcohol, methylparaben, and chlorocresol at the concentrations specified below shows only marginal effect on the Tₘ as demonstrated in Tables VII.

**Table VII.**

| Effect of pH, salt, and preservatives on Tₘ of uFSH in solution as monitored by DSC. | | |
|---|---|---|
| Solution conditions | | Tₘ (°C) |
| PB (9.5 mM phosphate) | | |
| | pH 5.7 | 72.4 |
| | pH 6.6 | 74.3 |
| | pH 7.6 | 74.8 |
| | pH 8.6 | 75.3 |
| | 100 mM NaCl, pH 7.6 | 76.1 |
| | | |
| PHS at pH 7.4 | | 77.3 |
| | 3.5 mg/ml *m* -Cresol | 75.3 |
| | 3.5 mg/ml Phenol | 75.0 |
| | 10 mg/ml Benzyl Alcohol | 73.5 |
| | 1 mg/ml Methyl Paraben | 76.1 |
| | 2 mg/ml Chlorocresol | 74.6 |

### Example 8

### Stability of uFSH as a Function of pH

Stability of uFSH (uFSH - Vitro Diagnostics - Human Urofollitropin) in PBS was determined for various pHs. Percent of heterodimer as a function of pH was monitored by size exclusion chromatography (SEC) in Table VIII.

**Table VIII.**

| Percent of heterodimer as a function of pH as monitored by SEC. | |
|---|---|
| pH | Percent Dimer |
| 7.0 | 95.0 |
| 6.5 | 95.0 |
| 6.0 | 95.0 |
| 5.5 | 95.0 |
| 5.0 | 95.0 |
| 4.5 | 95.0 |
| 4.0 | 95.0 |
| 3.5 | 95.0 |
| 3.25 | 87.7 |
| 3.0 | 62.3 |
| 2.5 | 17.6 |
| 2.0 | 5.0 |

### Reference EXAMPLE 9

### Stability of uFSH Preserved and Non-Preserved Formulations

A solution of uFSH (uFSH - Vitro Diagnostics - Human Urofollitropin) was prepared in PBS (Dulbecco's, GIBCO) and further diluted with PBS to a concentration of about 50 µg/ml. The protein concentration was determined on a Hewlett Packard Model 8452A Diode Array Spectrophotometer.

A portion of the uFSH solution was added to a beaker containing a pre-weighed sample of *m*-cresol to give a final *m*-cresol concentration of about 3.16 mg/ml. 1-ml aliquots of the preserved and non-preserved solution were placed in plastic centrifuge tubes and incubated up to 238 days at about 22°C, 37°C and 45°C. At various times, aliquots were injected onto a Superdex-75 HR 10/30 column (Pharmacia) equilibrated and run at ambient temperature at 0.5 ml/min. in PBS. The eluant was monitored at 214 nm. The percentage of the heterodimer was calculated from the ratio of the area of dimer peak divided by the total area of dimer peak and monomer peaks, as shown in Table III. After 64 days, about 79% of the uFSH molecules in solution with *m*-cresol remain as intact dimer at 37°C and more than 52 % stay as dimer at 45°C. Surprisingly, after 63 days at room temperature, there is minimal dissociation of uFSH heterodimer in both the non-preserved and preserved solutions. It is remarkable that at 23°C, 37°C, and 45°C there is generally relatively low dissociation of uFSH heterodimer in both non-preserved and preserved solutions at days 4, 8, 16, 21, 28, 29, 43, 63, 64, 126, 127, 237, and 238.

**Table IX. Percent Dimer in Preserved and Non-preserved uFSH solutions.**

| | PBS | | | PBS + *m*-cresol | | |
|---|---|---|---|---|---|---|
| Days | 23°C | 37°C | 45°C | 23°C | 37°C | 45°C |
| 0 | 96.0 | 96.0 | 96.0 | 93.8 | 93.8 | 93.8 |
| 4 | - | 93.0 | 91.7 | - | 91.8 | 85.6 |
| 8 | 94.3 | 92.7 | 89.5 | 93.6 | 88.9 | 80.0 |
| 16 | 94.1 | 91.1 | 84.3 | 92.5 | 87.0 | 73.8 |
| 21 | 94.0 | 91.0 | 83.1 | - | - | - |
| 22 | - | - | - | 92.3 | 84.7 | 68.8 |
| 28 | 92.8 | 89.8 | - | 92.7 | 83.7 | - |
| 29 | - | - | 79.9 | - | - | 65.3 |
| 43 | 93.3 | 89.5 | 75.9 | 91.6 | 81.5 | 59.2 |
| 63 | 92.9 | - | - | 92.6 | - | - |
| 64 | - | 88.2 | 68.6 | - | 78.9 | 52.5 |
| 126 | 91.5 | 85.5 | - | 89.6 | 71.2 | - |
| 127 | - | - | 58.2 | - | - | 43.8 |
| 237 | 92.3 | 83.1 | - | 91 | 62.0 | - |
| 238 | - | - | 57.9 | - | - | 39.2 |

### Reference EXAMPLE 10

### Bioactivity Measurements of uFSH Samples

HEK 293 cells stably transfected with a cAMP sensitive b-lactamase (BLAM) reporter vector (Zlokarnik, et al., 1998, Science 279:84-88) were transfected with a human FSH receptor expression vector encoding a hygromycin selectable marker and incubated in hygromycin for 3 weeks. The surviving cells were treated with 10 µg/ml of FSH (Sigma) for 5 hours and the population of FSH activated cells showing the highest intensity of blue fluorescence were identified and isolated by FACS. This polyclonal population was expanded, treated with 10 µg/ml of FSH for 5 hours, and FACS sorted into single cell clones. Two clonal cell lines were analyzed by the BLAM microtiter plate assay and showed a 6 to 8 fold increase in blue/green ratio. The FSH-R cell line with the greatest fold increase in BLAM expression was chosen as the cell line to be used in the subsequent FSH assays.

The FSH receptor cell line harboring the CAMP sensitive BLAM reporter was seeded in 100 µl Growth Medium (DMEM catalog number 11965-092, 10% FBS, 500 µg/ml Gentamicin) at 20,000 cells/well in a poly-D-lysine coated, 96-well black wall tissue culture plate, and incubated overnight at 37°C under 5% CO₂. The Growth Medium was replaced by 100 µl Assay Medium (DMEM catalog number 11965-092, 0.5% FBS, 500 µg/ml Gentamicin) and the plate was incubated overnight at 37°C and 5% CO2. The Assay Medium was removed and 100 µl of Assay Medium containing the indicated concentration of the FSH was then added to each well and the plate was incubated for 5 hours at 37°C under 5% CO₂. 20 µl of the BLAM substrate loading, composed of 6 µl of 1 mM CCF2-AM in DMSO, 6 µl Pluronic Acid (100 µg/ml in 0.1% acetic acid DMSO) into 1 ml 2% PEG-400 and 10% ESS (Aurora Biosciences) was then added into each well. After 1 hour of incubation at room temperature, the ratio of blue (395 nm excitation/460 nm emission) to green (395 nm excitation/530 nm emission) fluorescence intensities was determined with a Cytofluor (Perseptives Biosystems, Series 4000 multi-well plate reader). The fold increase in blue/green ratio resulting from the presence of FSH was calculated by dividing each ratio by the blue/green ratio of the control sample.

A solution of urinary FSH (uFSH - Vitro Diagnostics - Human Urofollitropin)at 50 µg/ml in PBS (Sample A) was prepared as in Example 9. A portion of this solution was heated at 90°C for 10 minutes to dissociate more than 99% of the heterodimer into the two monomers (as shown by SEC analysis) and was used in this assay (Sample B) as a negative control. Another portion of the FSH solution was modified to include about 3.15 mg/ml of m-cresol (Sample C). The bioactivity of these three test samples was evaluated in the FSH-R 293-Cre-BLAM assay on two separate plates. The average of triplicate analyses on each plate is shown in Table X. This data shows the assay was performing very reproducibly. It also showed that the dissociated heterodimer lost bioactivity (Sample B) and that the FSH in the formulation containing the m-cresol (Sample C) retained full bioactivity.

**Table X.**

| Blue/Green fold increase in the FSH receptor bioactivity assay. | | | | | | |
|---|---|---|---|---|---|---|
| Test Sample concentration (nM) | Sample A Plate 1 | Sample A Plate 2 | Sample B Plate 1 | Sample B Plate 2 | Sample C Plate 1 | Sample C Plate 2 |
| 0 (control) | 1.00 | 1.00 | 1.00 | 1.00 | 1.00 | 1.00 |
| 0.003 | 1.68 | 1.67 | 1.07 | 1.06 | 1.22 | 1.22 |
| 0.01 | 1.91 | 1.86 | 1.06 | 1.06 | 1.58 | 1.62 |
| 0.03 | 3.00 | 2.89 | 1.06 | 1.06 | 2.72 | 3.21 |
| 0.1 | 4.00 | 4.09 | 1.18 | 1.18 | 3.86 | 3.85 |
| 0.3 | 4.53 | 4.47 | 1.24 | 1.24 | 4.61 | 4.63 |
| 1 | 4.88 | 4.71 | 1.68 | 1.69 | 4.81 | 4.77 |
| 3 | 4.60 | 4.64 | 2.61 | 2.58 | 4.83 | 4.92 |

### Reference EXAMPLE 11

### Bioactivity Measurements of Preserved and Non-Preserved uFSH Samples

Bioactivity measurements of urinary FSH(uFSH - Vitro Diagnostics - Human Urofollitropin) samples were determined in the in vitro bioactivity assay as described in Example 10. In vitro assay results of two samples uFSH in PBS at 23 °C, PBS with and without m-cresol at 23 °C after 11 month compared to a control in table XI. The data indicates that preserved and non-preserved uFSH samples maintain full biological activity after 11 months in this assay.

**Table XI:**

| In vitro bioactivity of preserved and non-preserved uFSH solution after 11 month at 23 °C. | |
|---|---|
| Sample | Relative potency |
| uFSH control (50 µg/ml, PBS, fresh) | 1.0 |
| uFSH (50 µg/ml, PBS, 23 °C for 11 months) | 1.27 |
| uFSH (50 µg/ml, PBS, 3.15 mg/ml m-cresol, 23°C for 11 months) | 0.86 |

### Reference EXAMPLE 12

### Cartridge Compatibility of Preserved and Non-preserved rFSH Variant

To test the compatibility of formulated rFSH variant (alpha subunit SEQ ID NO:5; beta subunit SEQ ID NO:11) solutions with cartridges, 4-ml solutions of the samples as listed in Table 12 were prepared from stock solutions listed below:
1.85 mg/ml rFSH variant in PBS
1.73 mg/ml rFSH variant in PB
20 mg/ml m-cresol in PBS
20 mg/ml m-cresol in PB
20% glycerol in PB
1 x PBS

After mixing, 1.7 ml of each solution was pipetted into individual cartridges with minimal head-space allowed. Two cartridges were filled for each sample. Caps were sealed on the cartridges. The cartridges were then incubated at 30°C for 20 days. After filling, the remainder of the samples were incubated at 40°C to serve as control samples.

In vitro activity of these samples was measured after 20 days of incubation at 30°C, using the method described in Example 11. The activity of these samples was compared to corresponding control samples at zero time point. As shown in Table XII below, rFSH sample at 50 µg/ml in PBS and sample at 200 µg/ml in PBS and 3.15 mg/ml m-cresol are stable in cartridges (cartridge 1 and cartridge 4). These samples remain fully active after 20 days of incubation at 30°C. However, samples in phosphate buffer without NaCl were less potent under these conditions. The activity of rFSH samples in phosphate buffer and 1.6 % glycerol decreases in comparison to that of control (cartridge 2 and cartridge 3)

**Table XII.**

| In vitro activity of preserved and non-preserved samples incubated at 30 °C for 20 days in cartridges. | | |
|---|---|---|
| **Sample** | **Sample Conditions** | **Relative Potency** |
| Cartridge 1 | 200 µg/ml rFSH variant, 3.15 mg/ml *m*-cresol, PBS, pH 7.4 | 1.06 |
| Cartridge 2 | 200 µg/ml rFSH variant, 1.6% glycerol, PB, pH 7.4 | 0.81 |
| Cartridge 3 | 50 µg/ml rFSH variant, 1.6% glycerol, 3.15 mg/ml *m*-cresol, PB, pH 7.4 | 0.60 |
| Cartridge 4 | 50 µg/ml rFSH variant, PBS, pH 7.4 | 1.10 |

As demonstrated by these examples, following the methods and techniques described one can generate surprisingly stable composition and formulations of FSH or a FSH variant. These compositions and formulations result in the development of the presently claimed articles of manufacture.

The principles, preferred embodiments, and modes of operation of the present invention have been described in the foregoing specification. The invention intended to be protected herein, however, is not to be construed as limited to the particular forms disclosed, since they are to be regarded as illustrative rather than restrictive. Variations and changes may be made by those skilled in the art without departing from the spirit of the invention.

### EXAMPLE 13

### Formulation Stability of Preserved and Non-Preserved FSH Variant Samples

A stock solution of recombinant FSH variant (alpha subunit is SEQ ID:5; beta subunit SEQ ID:11) at about 1 mg/ml in phosphate buffered saline (PBS, Dulbecco's, GIBCO) was diluted to 50 µg/ml or 20 µg/ml with either PBS or PBS containing m-cresol to give a final m-cresol concentration of 3.15 mg/ml. Similarly, another set of samples was made using 10 mg/ml benzyl alcohol as preservative. 1-ml aliquots of the preserved and non-preserved solution were incubated at 4, 22, 37 °C for up to three months in plastic eppendorf tubes. At various times, aliquots were injected onto a Superdex 75 gel filtration column (Pharmacia) equilibrated with PBS and run at ambient temperature with a flow rate of 0.07 ml/min and the run time of 35 minutes. Detection was monitored by UV absorbance at 214 nm over time. Peak areas were integrated, and the percentage of the heterodimer was calculated as a ratio of area of heterodimer peak over the total area of the dimer and monomer peaks.
As shown in Table XIII, there is minimal dissociation of the heterodimer under various solution conditions after three month of incubation at room temperature or below. Greater than 50% heterodimer remains intact after three month incubation at 37°C. The stability of heterodimer is higher with more concentrated solution.

**Table XIII.**

| Heterodimer stability of rFSH variant monitored by SE-HPLC. | | | | | | |
|---|---|---|---|---|---|---|
| Sample | %Dimer | | | | | |
| | 1 month | | | 3 months | | |
| | 4°C | 22°C | 37°C | 4°C | 22°C | 37°C |
| 20 µg/ml in PBS | 100 | 100 | 88.9 | 100 | 100 | 77.3 |
| 20 µg/ml in PBS | 100 | 100 | 86.2 | 100 | 100 | 64.6 |
| 3.15 mg/ml m-cresol | | | | | | |
| 20 µg/ml in PBS | 100 | 100 | 89.1 | 100 | 100 | 57.1 |
| 10 mg/ml benzyl alcohol | | | | | | |
| 50 µg/ml in PBS | 100 | 100 | 100 | 100 | 100 | 81.1 |
| 50 µg/ml in PBS | 100 | 100 | 90 | 100 | 100 | 75.7 |
| 3.15 mg/ml m-cresol | | | | | | |
| 50 µg/ml in PBS | 100 | 100 | 87 | 100 | 100 | 61.0 |
| 10 mg/ml benzyl alcohol | | | | | | |

## Claims

1. A formulation comprising FSH or an FSH variant, containing an alpha and beta subunit, and a preservative which is benzyl alcohol in an aqueous diluent, wherein the formulation has a pH between 6.8 and 9 and is a multi-use formulation in a single solution vial for therapeutic use.

2. A formulation of Claim 1, wherein said FSH or FSH variant is at least one compound selected from the group consisting of:
(a): α-subunit:(SEQ ID NO:1) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
 β -subunit:(SEQ ID NO:2) 
RSCELTNITITVEKEECGFCISINTTWCAGYCYTRDLVYRDPARPNIQKTCTFKEL
VYETVKVPGCAHHADSLYTYPVATECHCSKCDSDSTDCTVRGLGPSYCSFREIKE
(b) :
α-subunit:(SEQ ID NO:3) 
FPDGEFTTQDCPECKLRENKYFFKLGVPIYQCKGCCFSRAYPTPARSRKTMLVPKN
ITSESTCCVAKAFIRVTVMGNIKLENHTQCYCSTCYHHKI
β-subunit:(SEQ ID NO:4) 
NSCELTNITIAVEKEGCGFCITINTTWCAGYCYTRDLVYKDPARPNIQKTCTFKEL
VYETVKVPGCAHHADSLYTYPVATACHCGKCNSDSTDCTVRGLGPSYCSFGDMKE
(c) :
α-subunit:(SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
β-subunit:(SEQ ID NO:6) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMKE
(d) :
α-subunit:(SEQ ID NO:7) 
FPDGEFTMQGCPECKLKENKYFSKLGAPIYQCMGCCFSRAYPTPARSKKTMLVPKN
ITSEATCCVAKAFTKATVMGNARVENHTECHCSTCYYHKS
β-subunit:(SEQ ID NO:8) 
NSCELTNITITVEKEECNFCISINTTWCAGYCYTRDLVYKDPARPNIQKTCTFKEL
VYETVKVPGCAHHADSLYTYPVATECHCGKCDSDSTDCTVRGLGPSYCSFSEMKE
(e) :
α-subunit:(SEQ ID NO:9) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
β-subunit:(SEQ ID NO:10) 
RSCELTNITITVEKEECSFCISINTTWCAGYCYTRDLVYKDPARPNIQKACTFKEL
VYETVKVPGCAHHADSLYTYPVATECHCGKCDRDSTDCTVRGLGPSYCSFSDIRE
(f) :
α-subunit:(SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
β-subunit:(SEQ ID NO:11) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGE
(g) :
α-subunit:(SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
β-subunit:(SEQ ID NO:12) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEM
(h) :
α-subunit:(SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
β-subunit:(SEQ ID NO:13)

NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMK

3. A formulation of Claims 1 or 2, wherein said FSH or FSH variant is human FSH.

4. A formulation of Claims 1-3, wherein said FSH or FSH variant is produced through the use of recombinant DNA technology.

5. A formulation of Claims 1-4, wherein said FSH or FSH variant is at a concentration of 5.0 µg/ml to 200 µg/ml.

6. A formulation of Claim 5, wherein said FSH or FSH variant is at a concentration of 50 µg/ml to 200 µg/ml.

7. A formulation of Claims 1-6, further comprising a physiologically acceptable buffer.

8. A formulation of Claim 7, wherein said buffer is a phosphate buffer with saline or an acceptable salt.

9. An article of manufacture comprising a single solution vial comprising a solution of FSH or an FSH variant, containing an alpha and beta subunit, a preservative which is benzyl alcohol, and an aqueous diluent; and packaging material comprising a label which indicates the solution may be held over a period of 24 hours or greater, wherein the solution has a pH between 6.8 and 9 and is a multi-use formulation for therapeutic use.

10. An article of manufacture of Claim 9, wherein said FSH or FSH variant is at least one compound selected from the group consisting of:
(a): α-subunit:(SEQ ID NO:1) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
 β -subunit:(SEQ ID NO:2) 
RSCELTNITITVEKEECGFCISINTTWCAGYCYTRDLVYRDPARPNIQKTCTFKEL
VYETVKVPGCAHHADSLYTYPVATECHCSKCDSDSTDCTVRGLGPSYCSFREIKE
(b) :
α-subunit:(SEQ ID NO:3) 
FPDGEFTTQDCPECKLRENKYFFKLGVPIYQCKGCCFSRAYPTPARSRKTMLVPKN
ITSESTCCVAKAFIRVTVMGNIKLENHTQCYCSTCYHHKI
β-subunit:(SEQ ID NO:4) 
NSCELTNITIAVEKEGCGFCITINTTWCAGYCYTRDLVYKDPARPNIQKTCTFKEL
VYETVKVPGCAHHADSLYTYPVATACHCGKCNSDSTDCTVRGLGPSYCSFGDMKE
(c) :
α-subunit:(SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
β-subunit:(SEQ ID NO:6) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMKE
(d) :
α-subunit:(SEQ ID NO:7) 
FPDGEFTMQGCPECKLKENKYFSKLGAPIYQCMGCCFSRAYPTPARSKKTMLVPKN
ITSEATCCVAKAFTKATVMGNARVENHTECHCSTCYYHKS
β-subunit:(SEQ ID NO:8) 
NSCELTNITITVEKEECNFCISINTTWCAGYCYTRDLVYKDPARPNIQKTCTFKEL
VYETVKVPGCAHHADSLYTYPVATECHCGKCDSDSTDCTVRGLGPSYCSFSEMKE
(e) :
α-subunit:(SEQ ID NO:9) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
β-subunit:(SEQ ID NO:10) 
RSCELTNITITVEKEECSFCISINTTWCAGYCYTRDLVYKDPARPNIQKACTFKEL
VYETVKVPGCAHHADSLYTYPVATECHCGKCDRDSTDCTVRGLGPSYCSFSDIRE
(f) :
α-subunit:(SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
β-subunit:(SEQ ID NO:11) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGE
(g):
α-subunit:(SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
β-subunit:(SEQ ID NO:12) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEM
(h) :
α-subunit:(SEQ ID NO:5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
β-subunit:(SEQ ID NO:13)

NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMK

11. An article of manufacture of Claims 9-10, wherein said FSH or FSH variant is human FSH.

12. An article of manufacture of Claims 9-11, wherein said FSH or FSH variant is produced through the use of recombinant DNA technology.

13. An article of manufacture of Claims 9-12, wherein said FSH or FSH variant is at a concentration of 5.0 µg/ml to 200 µg/ml.

14. An article of manufacture of Claim 13, wherein said FSH or FSH variant is at a concentration of 50 µg/ml to 200 µg/ml.

15. An article of manufacture of Claims 9-14, further comprising a physiologically acceptable buffer.

16. An article of manufacture of Claim 15, wherein said buffer is a phosphate buffer with saline or an acceptable salt.

17. An article of manufacture of Claims 9-16, wherein the solution may be held or used over a period of 96 hours or greater.

18. Use of a formulation of Claims 1-8 in the manufacture of a medicament for the treatment of infertility in women and/or men.

19. Use of a solution of FSH with benzyl alcohol as a preservative in the manufacture of a medicament for the treatment of infertility, said solution being a multi-use formulation in a single solution vial suitable for administration over a period of 96 hours or greater and having a pH between 6.8 and about 9.

## Patentansprüche

1. Formulierung, die FSH oder eine FSH Variante umfaßt, welche eine alpha- oder beta-Untereinheit enthält, und ein Konservierungsstoff, welcher Benzylalkohol in einem wäßrigen Verdünnungsmittel ist, wobei die Formulierung einen pH-Wert zwischen 6,8 und 9 hat und eine mehrfach anwendbare Formulierung in einem einzelnen Gläschen mit einer Lösung für therapeutische Verwendung ist.

2. Formulierung nach Anspruch 1, worin das FSH oder die FSH Variante zumindest eine Verbindung ist, die aus der Gruppe ausgewählt ist, welche besteht aus
(a) α-Untereinheit: (SEQ ID Nr. 1) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
 β -Untereinheit: (SEQ ID Nr. 2) 
RSCELTNITITVEKEECGFCISINTTWCAGYCYTRDLVYRDPARPNIQKTCTFKEL
VYETVKVPGCAHHADSLYTYPVATECHCSKCDSDSTDCTVRGLGPSYCSFREIKE
(b) α-Untereinheit: (SEQ ID Nr. 3) 
FPDGEFTTQDCPECKLRENKYFFKLGVPIYQCKGCCFSRAYPTPARSRKTMLVPKN
ITSESTCCVAKAFIRVTVMGNIKLENHTQCYCSTCYHHKI
 β -Untereinheit: (SEQ ID Nr. 4) 
NSCELTNITIAVEKEGCGFCITINTTWCAGYCYTRDLVYKDPARPNIQKTCTFKEL
VYETVKVPGCAHHADSLYTYPVATACHCGKCNSDSTDCTVRGLGPSYCSFGDMKE
(c) α-Untereinheit: (SEQ ID Nr. 5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -Untereinheit: (SEQ ID Nr. 6) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMKE
(d) α-Untereinheit: (SEQ ID Nr. 7) 
FPDGEFTMQGCPECKLKENKYFSKLGAPIYQCMGCCFSRAYPTPARSKKTMLVPKN
ITSEATCCVAKAFTKATVMGNARVENHTECHCSTCYYHKS
 β -Untereinheit: (SEQ ID Nr. 8) 
NSCELTNITITVEKEECNFCISINTTWCAGYCYTRDLVYKDPARPNIQKTCTFKEL
VYETVKVPGCAHHADSLYTYPVATECHCGKCDSDSTDCTVRGLGPSYCSFSEMKE
(e) α-Untereinheit: (SEQ ID Nr. 9) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
 β -Untereinheit: (SEQ ID Nr. 10) 
RSCELTNITITVEKEECSFCISINTTWCAGYCYTRDLVYKDPARPNIQKACTFKEL
VYETVKVPGCAHHADSLYTYPVATECHCGKCDRDSTDCTVRGLGPSYCSFSDIRE
(f) α-Untereinheit: (SEQ ID Nr. 5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -Untereinheit: (SEQ ID Nr. 11) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGE
(g) α-Untereinheit: (SEQ ID Nr. 5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -Untereinheit: (SEQ ID Nr. 12) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEM
(h) α-Untereinheit: (SEQ ID Nr. 5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -Untereinheit: (SEQ ID Nr. 13)

NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMK

3. Formulierung nach einem der Ansprüche 1 oder 2, worin das FSH oder die FSH Variante humanes FSH ist.

4. Formulierung nach einem der Ansprüche 1 bis 3, worin das FSH oder die FSH Variante durch die Verwendung von rekombinanter DNA Technologie hergestellt wird.

5. Formulierung nach einem der Ansprüche 1 bis 4, worin das FSH oder die FSH Variante in einer Konzentration von 5,0 µg/ml bis 200 µg/ml vorhanden ist.

6. Formulierung nach Anspruch 5, worin das FSH oder die FSH Variante in einer Konzentration von 50 µg/ml bis 200 µg/ml vorhanden ist.

7. Formulierung nach einem der Ansprüche 1 bis 6, die ferner einen physiologisch annehmbaren Puffer enthält.

8. Formulierung nach Anspruch 7, worin der Puffer ein Phosphatpuffer mit Kochsalzlösung oder einem annehmbaren Salz ist.

9. Serienartikel, der ein einzelnes Gläschen mit einer Lösung, das eine Lösung an FSH oder einer FSH Variante, welche eine alpha- oder beta-Untereinheit enthält, einen Konservierungsstoff, welcher Benzylalkohol ist, und ein wäßriges Verdünnungsmittel umfaßt und ein Verpackungsmaterial, das eine Aufschrift aufweist, die anzeigt, daß die Lösung für einen Zeitraum von 24 Stunden oder länger aufbewahrt werden kann, wobei die Lösung einen pH-Wert zwischen 6,8 und 9 hat und eine mehrfach anwendbare Formulierung für therapeutische Verwendung ist.

10. Serienartikel nach Anspruch 9, worin das FSH oder die FSH Variante zumindest eine Verbindung ist, die aus der Gruppe ausgewählt ist, welche besteht aus
(a) α-Untereinheit: (SEQ ID Nr. 1) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
 β -Untereinheit: (SEQ ID Nr. 2) 
RSCELTNITITVEKEECGFCISINTTWCAGYCYTRDLVYRDPARPNIQKTCTFKEL
VYETVKVPGCAHHADSLYTYPVATECHCSKCDSDSTDCTVRGLGPSYCSFREIKE
(b) α-Untereinheit: (SEQ ID Nr. 3) 
FPDGEFTTQDCPECKLRENKYFFKLGVPIYQCKGCCFSRAYPTPARSRKTMLVPKN
ITSESTCCVAKAFIRVTVMGNIKLENHTQCYCSTCYHHKI
 β -Untereinheit: (SEQ ID Nr. 4) 
NSCELTNITIAVEKEGCGFCITINTTWCAGYCYTRDLVYKDPARPNIQKTCTFKEL
VYETVKVPGCAHHADSLYTYPVATACHCGKCNSDSTDCTVRGLGPSYCSFGDMKE
(c) α-Untereinheit: (SEQ ID Nr. 5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -Untereinheit: (SEQ ID Nr. 6) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMKE
(d) α-Untereinheit: (SEQ ID Nr. 7) 
FPDGEFTMQGCPECKLKENKYFSKLGAPIYQCMGCCFSRAYPTPARSKKTMLVPKN
ITSEATCCVAKAFTKATVMGNARVENHTECHCSTCYYHKS
 β -Untereinheit: (SEQ ID Nr. 8) 
NSCELTNITITVEKEECNFCISINTTWCAGYCYTRDLVYKDPARPNIQKTCTFKEL
VYETVKVPGCAHHADSLYTYPVATECHCGKCDSDSTDCTVRGLGPSYCSFSEMKE
(e) α-Untereinheit: (SEQ ID Nr. 9) 
FPDGEFTMQGCPECKLKENKYFSKPDAPIYQCMGCCFSRAYPTPARSKKTMLVPKN
ITSEATCCVAKAFTKATVMGNVRVENHTECHCSTCYYHKS
 β -Uniereinheit: (SEQ ID Nr. 10) 
RSCELTNITITVEKEECSFCISINTTWCAGYCYTRDLVYKDPARPNIQKACTFKEL
VYETVKVPGCAHHADSLYTYPVATECHCGKCDRDSTDCTVRGLGPSYCSFSDIRE
(f) α-Untereinheit: (SEQ ID Nr. 5) 
APDVQDCPECTLQENPFF SQPGAPILQCMGCCFSRAYPTPLRSKKTMLVQKNVTSE
STCCVAKSYNRVTVMGGFKVENHTACHCSTCYYHKS
 β -Untereinheit: (SEQ ID Nr. 11) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGE
(g) α-Untereinheit: (SEQ ID Nr. 5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -Untereinheit: (SEQ ID Nr. 12) 
NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEM
(h) α-Untereinheit: (SEQ ID Nr. 5) 
APDVQDCPECTLQENPFFSQPGAPILQCMGCCFSRAYPTPLRSKKTMLVQKNVTSE
STCCVAKSYNRVTVMGGFKVENHTACHCSTCYYHKS
 β -Untereinheit: (SEQ ID Nr. 13)

NSCELTNITIAIEKEECRFCISINTTWCAGYCYTRDLVYKDPARPKIQKTCTFKEL
VYETVRVPGCAHHADSLYTYPVATQCHCGKCDSDSTDCTVRGLGPSYCSFGEMK

11. Serienartikel nach einem der Ansprüche 9 bis 10, worin das FSH oder die FSH Variante humanes FSH ist.

12. Serienartikel nach einem der Ansprüche 9 bis 11, worin das FSH oder die FSH Variante durch die Verwendung von rekombinanter DNA Technologie hergestellt wird.

13. Serienartikel nach einem der Ansprüche 9 bis 12, worin das FSH oder die FSH Variante in einer Konzentration von 5,0 µg/ml bis 200 µg/ml vorhanden ist.

14. Serienartikel nach Anspruch 13, worin das FSH oder die FSH Variante in einer Konzentration von 50 µg/ml bis 200 µg/ml vorhanden ist.

15. Serienartikel nach einem der Ansprüche 9 bis 14, der ferner einen physiologisch annehmbaren Puffer enthält

16. Serienartikel nach Anspruch 15, worin der Puffer ein Phosphatpuffer mit Kochsalzlösung oder einem annehmbaren Salz ist.

17. Serienartikel nach einem der Ansprüche 9 bis 16, worin die Lösung für einen Zeitraum von 96 Stunden oder länger aufbewahrt oder verwendet werden kann.

18. Verwendung einer Formulierung nach einem der Ansprüche 1 bis 8 zur Herstellung eines Arzneimittels zur Behandlung von Unfruchtbarkeit bei Frauen und/oder Männern.

19. Verwendung einer FSH Lösung mit Benzylalkohol als Konservierungsstoff zur Herstellung eines Arzneimittels zur Behandlung von Unfruchtbarkeit, wobei die Lösung eine mehrfach anwendbare Formulierung in einem einzelnen Gläschen mit einer Lösung ist, die für die Verabreichung über einen Zeitraum von 96 Stunden oder mehr geeignet ist und einen pH-Wert zwischen 6,8 und etwa 9 hat.

## Revendications

1. Formulation comprenant de la FSH ou un variant de la FSH, contenant des sous-unités alpha et bêta, et un conservateur, à savoir de l'alcool benzylique dans un diluant aqueux, la formulation possédant un pH entre 6,8 et 9 et représentant une formulation polyvalente dans un flacon de solution unique pour une utilisation thérapeutique.

2. Formulation selon la revendication 1, dans laquelle ladite FSH ou ledit variant de la FSH représente au moins un composé choisi parmi le groupe constitué par :
(a) : sous-unité α : (SEQ ID NO: 1) 
FPDGEFTMQGCPECKLKENKYFSKPDAPIYQCMGCCFSRAYPTPARSKKTML
VPKNITSEATCCVAKAFTKATVMGNVRVENHTECHCSTCYYHKS
 sous-unité β : (SEQ ID NO: 2) 
RSCELTNITITVEKEECGFCISINTTWCAGYCYTRDLVYRDPARPNIQKTCTFKE
LVYETVKVPGCAHHADSLYTYPVATECHCSKCDSDSTDCTVRGLGPSYCSFR
EIKE
(b) : sous-unité α : (SEQ ID NO: 3) 
FPDGEFTTQDCPECKLRENKYFFKLGVPIYQCKGCCFSRAYPTPARSRKTML
VPKNITSESTCCVAKAFIRVTVMGNIKLENHTQCYCSTCYHHKI
 sous-unité β : (SEQ ID NO: 4) 
NSCELTNITIAVEKEGCGFCITINTTWCAGYCYTRDLVYKDPARPNIQKTCTFK
ELVYETVKVPGCAHHADSLYTYPVATACHCGKCNSDSTDCTVRGLGPSYCSF
GDMKE
(c) : sous-unité α : (SEQ ID NO: 5) 
APDVQDCPECTLQENPFFSQPGAPILQCMGCCFSRAYPTPLRSKKTMLVQKN
VTSESTCCVAKSYNRVTVMGGFKVENHTACHCSTCYYHKS
 sous-unité β : (SEQ ID NO: 6) 
NSCELTNITIAIEKEECRFCISINTTWCAGYCYTRDLVYKDPARPKIQKTCTFKE
LVYETVRVPGCAHHADSLYTYPVATQCHCGKCDSDSTDCTVRGLGPSYCSF
GEMKE
(d) : sous-unité α : (SEQ ID NO: 7) 
FPDGEFTMQGCPECKLKENKYFSKLGAPIYQCMGCCFSRAYPTPARSKKTML
VPKNITSEATCCVAKAFTKATVMGNARVENHTECHCSTCYYHKS
 sous-unité β : (SEQ ID NO: 8) 
NSCELTNITITVEKEECNFCISINTTWCAGYCYTRDLVYKDPARPNIQKTCTFKE
LVYETVKVPGCAHHADSLYTYPVATECHCGKCDSDSTDCTVRGLGPSYCSFS
EMKE
(e) : sous-unité α : (SEQ ID NO: 9) 
FPDGEFTMQGCPECKLKENKYFSKPDAPIYQCMGCCFSRAYPTPARSKKTML
VPKNITSEATCCVAKAFTKATVMGNVRVENHTECHCSTCYYHKS
 sous-unité β : (SEQ ID NO: 10) 
RSCELTNITITVEKEECSFCISINTTWCAGYCYTRDLVYKDPARPNIQKACTFKE
LVYETVKVPGCAHHADSLYTYPVATECHCGKCDRDSTDCTVRGLGPSYCSFS
DIRE
(f) : sous-unité α : (SEQ ID NO: 5) 
APDVQDCPECTLQENPFFSQPGAPILQCMGCCFSRAYPTPLRSKKTMLVQKN
VTSESTCCVAKSYNRVTVMGGFKVENHTACHCSTCYYHKS
 sous-unité β : (SEQ ID NO: 11) 
NSCELTNITIAIEKEECRFCISINTTWCAGYCYTRDLVYKDPARPKIQKTCTFKE
LVYETVRVPGCAHHADSLYTYPVATQCHCGKCDSDSTDCTVRGLGPSYCSF
GE
(g) : sous-unité α : (SEQ ID NO: 5) 
APDVQDCPECTLQENPFFSQPGAPILQCMGCCFSRAYPTPLRSKKTMLVQKN
VTSESTCCVAKSYNRVTVMGGFKVENHTACHCSTCYYHKS
 sous-unité β : (SEQ ID NO: 12) 
NSCELTNITIAIEKEECRFCISINTTWCAGYCYTRDLVYKDPARPKIQKTCTFKE
LVYETVRVPGCAHHADSLYTYPVATQCHCGKCDSDSTDCTVRGLGPSYCSF
GEM
(h) : sous-unité α : (SEQ ID NO: 5) 
APDVQDCPECTLQENPFFSQPGAPILQCMGCCFSRAYPTPLRSKKTMLVQKN
VTSESTCCVAKSYNRVTVMGGFKVENHTACHCSTCYYHKS
 sous-unité β : (SEQ ID NO: 13)

NSCELTNITIAIEKEECRFCISINTTWCAGYCYTRDLVYKDPARPKIQKTCTFKE
LVYETVRVPGCAHHADSLYTYPVATQCHCGKCDSDSTDCTVRGLGPSYCSF
GEMK.

3. Formulation selon la revendication 1 ou 2, dans laquelle ladite FSH ou ledit variant de la FSH est la FSH humaine.

4. Formulation selon les revendications 1 à 3, dans laquelle on produit ladite FSH ou ledit variant de la FSH en utilisant la technologie de l'ADN recombinant.

5. Formulation selon les revendications 1 à 4, dans laquelle ladite FSH ou ledit variant de la FSH est présent en une concentration de 5,0 µg/ml à 200 µg/ml.

6. Formulation selon la revendication 5, dans laquelle ladite FSH ou ledit variant de la FSH est présent en une concentration de 50 µg/ml à 200 µg/ ml.

7. Formulation selon les revendications 1 à 6, comprenant en outre un tampon physiologiquement acceptable.

8. Formulation selon la revendication 7, dans laquelle ledit tampon est un tampon de phosphate avec une solution saline ou encore un sel acceptable.

9. Article de fabrication comprenant un flacon comprenant un flacon de solution unique comprenant une solution de FSH ou d'un variant de la FSH, contenant des sous-unités alpha et bêta, un conservateur, à savoir de l'alcool benzylique, et un diluant aqueux, et une matière de conditionnement comprenant une étiquette qui indique que la solution peut être conservée pendant un laps de temps de 24 heures ou plus, la solution possédant un pH entre 6,8 et 9 et représentant une formulation polyvalente pour une utilisation thérapeutique.

10. Article de fabrication selon la revendication 9, dans lequel ladite FSH ou ledit variant de la FSH représente au moins un composé choisi parmi le groupe constitué par :
(a) : sous-unité α : (SEQ ID NO: 1) 
FPDGEFTMQGCPECKLKENKYFSKPDAPIYQCMGCCFSRAYPTPARSKKTML
VPKNITSEATCCVAKAFTKATVMGNVRVENHTECHCSTCYYHKS
 sous-unité β : (SEQ ID NO: 2) 
RSCELTNITITVEKEECGFCISINTTWCAGYCYTRDLVYRDPARPNIQKTCTFKE
LVYETVKVPGCAHHADSLYTYPVATECHCSKCDSDSTDCTVRGLGPSYCSFR
EIKE
(b) : sous-unité α : (SEQ ID NO: 3) 
FPDGEFTTQDCPECKLRENKYFFKLGVPIYQCKGCCFSRAYPTPARSRKTML
VPKNITSESTCCVAKAFIRVTVMGNIKLENHTQCYCSTCYHHKI
 sous-unité β : (SEQ ID NO: 4) 
NSCELTNITIAVEKEGCGFCITINTTWCAGYCYTRDLVYKDPARPNIQKTCTFK
ELVYETVKVPGCAHHADSLYTYPVATACHCGKCNSDSTDCTVRGLGPSYCSF
GDMKE
(c) : sous-unité α : (SEQ ID NO: 5) 
APDVQDCPECTLQENPFFSQPGAPILQCMGCCFSRAYPTPLRSKKTMLVQKN
VTSESTCCVAKSYNRVTVMGGFKVENHTACHCSTCYYHKS
 sous-unité β : (SEQ ID NO: 6) 
NSCELTNITIAIEKEECRFCISINTTWCAGYCYTRDLVYKDPARPKIQKTCTFKE
LVYETVRVPGCAHHADSLYTYPVATQCHCGKCDSDSTDCTVRGLGPSYCSF
GEMKE
(d) : sous-unité α : (SEQ ID NO: 7) 
FPDGEFTMQGCPECKLKENKYFSKLGAPIYQCMGCCFSRAYPTPARSKKTML
VPKNITSEATCCVAKAFTKATVMGNARVENHTECHCSTCYYHKS
 sous-unité β : (SEQ ID NO: 8) 
NSCELTNITITVEKEECNFCISINTTWCAGYCYTRDLVYKDPARPNIQKTCTFKE
LVYETVKVPGCAHHADSLYTYPVATECHCGKCDSDSTDCTVRGLGPSYCSFS
EMKE
(e) : sous-unité α : (SEQ ID NO: 9) 
FPDGEFTMQGCPECKLKENKYFSKPDAPIYQCMGCCFSRAYPTPARSKKTML
VPKNITSEATCCVAKAFTKATVMGNVRVENHTECHCSTCYYHKS
 sous-unité β : (SEQ ID NO: 10) 
RSCELTNITITVEKEECSFCISINTTWCAGYCYTRDLVYKDPARPNIQKACTFKE
LVYETVKVPGCAHHADSLYTYPVATECHCGKCDRDSTDCTVRGLGPSYCSFS
DIRE
(f) : sous-unité α : (SEQ ID NO: 5) 
APDVQDCPECTLQENPFFSQPGAPILQCMGCCFSRAYPTPLRSKKTMLVQKN
VTSESTCCVAKSYNRVTVMGGFKVENHTACHCSTCYYHKS
 sous-unité β : (SEQ ID NO: 11) 
NSCELTNITIAIEKEECRFCISINTTWCAGYCYTRDLVYKDPARPKIQKTCTFKE
LVYETVRVPGCAHHADSLYTYPVATQCHCGKCDSDSTDCTVRGLGPSYCSF
GE
(g) : sous-unité α : (SEQ ID NO: 5) 
APDVQDCPECTLQENPFFSQPGAPILQCMGCCFSRAYPTPLRSKKTMLVQKN
VTSESTCCVAKSYNRVTVMGGFKVENHTACHCSTCYYHKS
 sous-unité β : (SEQ ID NO: 12) 
NSCELTNITIAIEKEECRFCISINTTWCAGYCYTRDLVYKDPARPKIQKTCTFKE
LVYETVRVPGCAHHADSLYTYPVATQCHCGKCDSDSTDCTVRGLGPSYCSF
GEM
(h) : sous-unité α : (SEQ ID NO: 5) 
APDVQDCPECTLQENPFFSQPGAPILQCMGCCFSRAYPTPLRSKKTMLVQKN
VTSESTCCVAKSYNRVTVMGGFKVENHTACHCSTCYYHKS
 sous-unité β : (SEQ ID NO: 13)

NSCELTNITIAIEKEECRFCISINTTWCAGYCYTRDLVYKDPARPKIQKTCTFKE
LVYETVRVPGCAHHADSLYTYPVATQCHCGKCDSDSTDCTVRGLGPSYCSF
GEMK

11. Article de fabrication selon les revendications 9 à 10, dans lequel ladite FSH ou ledit variant de la FSH est la FSH humaine.

12. Article de fabrication selon les revendications 9 à 11, dans lequel on produit ladite FSH ou ledit variant de la FSH en utilisant la technologie de l'ADN recombinant.

13. Article de fabrication selon les revendications 9 à 12, dans lequel ladite FSH ou ledit variant de la FSH est présent en une concentration de 5,0 µg/ml à 200 µg/ml.

14. Article de fabrication selon la revendication 13, dans lequel ladite FSH ou ledit variant de la FSH est présent en une concentration de 50 µg/ml à 200 µg/ml.

15. Article de fabrication selon les revendications 9 à 14, comprenant en outre un tampon physiologiquement acceptable.

16. Article de fabrication selon la revendication 15, dans lequel ledit tampon est un tampon de phosphate avec une solution saline ou encore un sel acceptable.

17. Article de fabrication selon les revendications 9 à 16, dans lequel la solution peut être conservée ou utilisée pendant un laps de temps de 96 heures ou plus.

18. Utilisation d'une formulation selon les revendications 1 à 8 dans la préparation d'un médicament pour le traitement de la stérilité chez la femme et/ ou chez l'homme.

19. Utilisation d'une solution de FSH avec de l'alcool benzylique à titre de conservateur dans la préparation d'un médicament destiné au traitement de la stérilité, ladite solution représentant une formulation polyvalente dans un flacon de solution unique pour une administration pendant un laps de temps de 96 heures ou plus et possédant un pH entre 6,8 et environ 9.
